# EUROPEAN PATENT APPLICATION

(11) **EP 4 661 016 A1**
(43) Date of publication of application: **10.12.2025**
(21) Application number: 24750052.3
(22) Date of filing: 23.01.2024
(51) Int. Cl.: G16H 10/60

(54) **MEDICAL INFORMATION PROCESSING PROGRAM AND MEDICAL INFORMATION PROCESSING DEVICE**

(30) Priority: 03.02.2023 JP 2023015166; 03.02.2023 JP 2023015167; 03.02.2023 JP 2023015168; 05.06.2023 JP 2023092664; 05.06.2023 JP 2023092665
(71) Applicant: NIDEK CO., LTD., Gamagori-shi, Aichi 443-0038 (JP)
(72) Inventor: YOSHIKAWA, Yuichiro, Gamagori-shi Aichi 443-0038 (JP); MISHIMA, Tatsunori, Gamagori-shi Aichi 443-0038 (JP); MIYAGI, Tomohiro, Gamagori-shi Aichi 443-0038 (JP); TANAKA, Kiyoto, Gamagori-shi Aichi 443-0038 (JP); HOSHIKAWA, Yasuhiro, Gamagori-shi Aichi 443-0038 (JP); NORITAKE, Yosuke, Gamagori-shi Aichi 443-0038 (JP); HIBI, Kenji, Gamagori-shi Aichi 443-0038 (JP); HARIYAMA, Shinichiro, Gamagori-shi Aichi 443-0038 (JP); TAKEUCHI, Daisuke, Gamagori-shi Aichi 443-0038 (JP); CHADANI, Takumi, Gamagori-shi Aichi 443-0038 (JP); HASEGAWA, Takahiro, Gamagori-shi Aichi 443-0038 (JP); NONO, Yasuhiko, Gamagori-shi Aichi 443-0038 (JP); ISHIBASHI, Mariko, Gamagori-shi Aichi 443-0038 (JP); NAKAGAMI, Takashi, Gamagori-shi Aichi 443-0038 (JP); TORII, Hisanari, Gamagori-shi Aichi 443-0038 (JP); MAEYAMA, Takaya, Gamagori-shi Aichi 443-0038 (JP); MIZUNO, Yusuke, Gamagori-shi Aichi 443-0038 (JP)
(74) Representative: Viering, Jentschura & Partner mbB Patent- und Rechtsanwälte
(86) International application number: PCT/JP2024/001859
(87) International publication number: WO 2024/162100

(57) **Abstract**

The database is configured to store, for each patient, a plurality of types of medical information in association with the acquisition time of each piece of information. The medical information processing device identifies a target patient whose medical information is to be processed. The medical information processing device displays, as a matrix display on the display unit, at least some of the plurality of types of medical information concerning the target patient, by arranging the plurality of types of medical information by type in one of the vertical and horizontal rows and arranging the information by acquisition time in chronological order in the other of the vertical and horizontal rows.

## Description

### [Technical Field]

The present disclosure relates to a medical information processing program and a medical information processing device for processing medical information relating to patients.

### [Background Art]

A variety of techniques for improving the efficiency of treatment for patients have been proposed. For example, the medical information processing device described in Patent Literature1 facilitates prediction of disease progression by displaying, on a display unit, multiple medical images including medical images of a subject patient and medical images similar thereto, in a state in which the time axes of the imaging timings are aligned. Also, Patent Literature 1 discloses techniques for displaying graphs that show changes in the degree of disease progression.

### [Prior Art Literature]

### [Patent Literature]

[Patent Literature 1] WO 2021/066039 A1

### [Summary of Invention]

In recent years, with the progress of medical technology, the types and amounts of medical information (e.g., medical images, examination results, findings, prescriptions, treatment details, etc.) acquired for each patient have increased. As the types and amounts of medical information increase, it contributes to improvements in the accuracy of medical care. Meanwhile, medical professionals need to select and identify the medical information necessary for patient treatment from among the numerous types and large amounts of medical information. In such cases, for example, there is a risk that the efficiency of medical care may decrease, or that necessary medical information may be overlooked. Accordingly, technology capable of appropriately presenting multiple types of medical information to medical professionals is desired.

A typical objective of the present disclosure is to provide a medical information processing program and a medical information processing device capable of appropriately presenting multiple types of medical information to medical professionals.

A medical information processing program according to a typical embodiment of the present disclosure is executed in a medical information processing device that processes medical information concerning a patient, a database configured to store, for each patient, a plurality of types of medical information in association with acquisition time of each piece of information, the medical information processing program, when executed by a control unit of the medical information processing device, causing the medical information processing device to execute: a patient identification step of identifying a target patient whose medical information is to be processed; and a matrix display step of displaying, as a matrix display on a display unit, at least some of the plurality of types of medical information concerning the target patient by arranging the plurality of types of medical information by type in one of a vertical column and a horizontal row and by arranging the information by the acquisition time in chronological order in an other of the vertical column and the horizontal row.

A medical information processing device provided by a typical embodiment in the present disclosure is configured to process medical information concerning a patient, a database configured to store, for each patient, a plurality of types of medical information in association with acquisition time of each piece of information. The medical information processing device includes a control unit configured to execute: a patient identifying step of identifying a target patient whose medical information is to be processed; and a matrix display step of displaying, as a matrix display on a display unit, at least some of the plurality of types of medical information concerning the target patient by arranging the plurality of types of medical information by type in one of a vertical column or a horizontal row and by arranging the information by the acquisition time in chronological order in an other of the vertical column and the horizontal row.

According to the medical information processing program and medical information processing device according to the present disclosure, multiple types of medical information is appropriately presented to healthcare professionals.

### [General Content]

The medical information processing device exemplified in the present disclosure processes medical information relating to patients. The database is configured to store, for each patient, a plurality of types of medical information in association with the acquisition time of each piece of information. The control unit of the medical information processing device is configured to perform a patient identification step and a matrix display step. In the patient identification step, the control unit specifies a target patient whose medical information is to be processed. In the matrix display step, the control unit arranges at least some of the multiple types of medical information relating to the target patient along one axis by type, arranges them along the other axis in chronological order based on the times when the information was acquired, and displays the information as a matrix on a display unit.

According to the technology described in the present disclosure, multiple types of medical information relating to the target patient are displayed in a matrix format on a display unit according to each display order and chronological order. Therefore, multiple types of medical information relating to the target patient can be appropriately presented to medical professionals.

### (First aspect)

A first aspect of the medical information processing program and medical information processing device of the present disclosure will be described. The medical information processing device exemplified in the present disclosure processes medical information relating to patients. The database is configured to store, for each patient, a plurality of types of medical information in association with the acquisition time of each piece of information. The control unit of the medical information processing device is configured to perform a patient identification step, a display rule setting step, and a matrix display step. In the patient identification step, the control unit specifies a target patient whose medical information is to be processed. In the display rule setting step, the control unit sets at least one display rule from among a plurality of display rules, the display rules defining at least one of: rules for narrowing down the types of medical information to be displayed on the display unit from among multiple types of medical information relating to the target patient, and rules for determining the display order of the multiple types of medical information. In the matrix display step, the control unit executes, for the multiple types of medical information relating to the target patient, at least one of a type narrowing process and a display order determination process in accordance with the display rule set in the display rule setting step, then arranges the medical information in display order along one axis, and in chronological order of acquisition along the other axis, and displays them on the display unit in a matrix format.

According to the technology described in the present disclosure, a process for at least one of narrowing down the types of medical information to be displayed on the display unit and determining the display order (hereinafter, referred to as "display information adjustment processing") is executed for multiple types of medical information in accordance with a set display rule. The multiple types of medical information that have undergone display information adjustment processing are displayed on the display unit in a matrix format according to the display order and chronological order. Therefore, from among a wide variety and large amount of medical information, partial or all medical information selected in accordance with the display rule (partial or the entire medical information) is displayed in an appropriate display order in a matrix format. As a result, necessary medical information is appropriately presented to medical professionals.

Various types of medical information (for example, at least some of medical images, examination results, findings, symptoms, prescriptions, treatment details, surgical details, etc.) may be comprised in the multiple types of medical information.

The control unit may execute a display rule generation step in which display rules are generated based on instructions input by a user and stored in the database. In this case, medical professionals can pre-generate display rules so that the medical information they wish to view is displayed in a matrix format in an appropriate display order. Thus, necessary medical information can be presented more appropriately to medical professionals.

The method for generating display rules can be selected as appropriate. For example, at least some of the plurality of display rules may be pre-generated. Additionally, new display rules may be generated by appropriately modifying pre-generated display rules based on instructions input by the user.

In the display rule setting step, the control unit may set at least one display rule from among a plurality of display rules in accordance with instructions input by a user. In this case, medical professionals can view the medical information they desire in a matrix format arranged in an appropriate display order. Thus, necessary medical information can be presented more appropriately to medical professionals.

Among the plurality of display rules, at least one of the rules for narrowing down types of medical information and for determining display order may comprise multiple display rules defined for each of a plurality of diseases. In the display rule setting step, the control unit may set display rules corresponding to the disease of the target patient. When treating patients, the importance of each type of medical information often varies depending on the patient's disease. Accordingly, when medical information is displayed in a matrix format on the display unit in accordance with display rules that correspond to the disease of the target patient, it becomes easier for medical professionals to appropriately treat the disease based on the displayed medical information.

The control unit may further execute a disease information acquisition step for acquiring disease information relating to the disease of the target patient. In the display rule setting step, the control unit may automatically set display rules corresponding to the disease indicated by the disease information of the target patient acquired in the disease information acquisition step. In this case, medical information is automatically displayed on the display unit in accordance with the display rules corresponding to the disease of the target patient. Therefore, the efficiency of medical care by medical professionals can be further improved.

In the disease information acquisition step, the control unit may acquire disease information of the target patient by inputting medical information of the target patient into a mathematical model trained using a machine learning algorithm. The mathematical model may be trained to output an automatic determination result for the disease of the target patient based on the input medical information. In this case, even if the medical professional does not input the disease information of the target patient manually, the disease information can be appropriately acquired based on the automatic determination result output from the mathematical model. Thus, it becomes easier to further improve the efficiency of medical care by medical professionals.

Specific methods for acquiring disease information of the target patient can be selected as appropriate. For example, the control unit may automatically acquire information indicating the disease of the target patient from information about the target patient stored in a database (for example, electronic medical record information, reservation information when the patient makes a medical appointment, etc.). Also, in order to provide medical care for specific diseases, specific tests or examinations may be performed. In this case, information indicating the disease of the target patient may be automatically acquired based on the type and number of tests or examinations performed on the target patient.

### (Second aspect)

A second aspect of the medical information processing program and medical information processing device of the present disclosure will be described. The medical information processing device exemplified in the present disclosure processes medical information relating to patients. The database is configured to store, for each patient, a plurality of types of medical information in association with the acquisition time of each piece of information. The control unit of the medical information processing device is configured to perform a patient identification step, a display rule setting step, and a matrix display step. In the patient identification step, the control unit specifies a target patient whose medical information is to be processed. In the display rule setting step, the control unit sets display rules for at least one disease from among a plurality of display rules, the display rules defining at least one of: rules for narrowing down the types of medical information to be displayed on the display unit from among multiple types of medical information relating to the target patient, and rules for determining the display order of the multiple types of medical information according to each of the multiple diseases. In the matrix display step, the control unit executes, for the multiple types of medical information relating to the target patient, at least one of a type narrowing process and a display order determination process in accordance with the display rule for the disease set in the display rule setting step, then arranges the medical information in display order along one axis, and arranges them along the other axis in chronological order of acquisition, and displays them on the display unit in a matrix format.

According to the technology described in the present disclosure, a process for at least one of narrowing down the types of medical information to be displayed on the display unit and determining the display order (hereinafter, referred to as "display information adjustment processing") is executed for multiple types of medical information in accordance with the set disease display rules. Multiple types of medical information that have undergone display information adjustment processing are displayed in a matrix format on a display unit according to the display order and chronological order (hereinafter, referred to as a "matrix display"). Therefore, from among the wide variety and large amount of medical information, partial or all medical information determined by the display rule corresponding to the disease is displayed in an appropriate display order in a matrix format. Thus, medical information necessary for treating the disease of the target patient can be appropriately presented to medical professionals.

Various types of medical information (for example, at least some of medical images, examination results, findings, symptoms, prescriptions, treatment details, surgical details, etc.) may be comprised in the multiple types of medical information.

The control unit may further execute a disease history information display step in which disease history information showing the chronological course of a specific disease of the target patient is displayed on the display unit based on medical information relating to the specific disease of the target patient. In this case, in addition to being able to appropriately identify multiple types of medical information through the matrix display, medical professionals can also easily identify the course of the specific disease using the disease history information. Therefore, the efficiency and accuracy of patient treatment can be further improved.

In the disease history information display step, the control unit may display information indicating that an event relating to the specific disease of the target patient has occurred, along with the time period when the event occurred, in the disease history information (for example, by displaying an icon mark, etc.). In this case, medical professionals can appropriately identify the period in which an event relating to the specific disease of the target patient occurred by using disease history information showing the progression of the disease over time. Therefore, the efficiency and accuracy of patient treatment can be further improved.

The content of event information to be displayed in the disease history information can be selected as appropriate. For example, at least any of the following may be displayed in the disease history information by using icons or other means: a hospital visit event indicating that the target patient visited the hospital; a surgical event indicating that surgery was performed on the target patient; a prescription event indicating that a prescription was issued for the target patient; an examination event indicating that an examination (including imaging) was performed on the target patient; and a test result event indicating that the results of tests repeatedly performed on the target patient satisfy a predetermined condition (for example, a condition where test results exceed a threshold value, or a condition where the amount of change in test results exceeds a threshold value). For example, by displaying test result events in the disease history information, medical professionals can appropriately identify the time period when the test results became noteworthy.

The disease history information display step may comprise a related disease history information display step in which disease history information relating to a related disease occurring in connection with a specific disease is displayed branching off from the disease history information of the specific disease. In this case, medical professionals can appropriately identify the circumstances of related diseases occurring in connection with the specific disease (hereinafter, also referred to as the "underlying disease") by comparing them with the course of the underlying disease.

Specific methods for displaying the related disease history information can be selected as appropriate. For example, the control unit may branch and display the related disease history information from the disease history information of the underlying disease in accordance with instructions input by a user. The correspondence relationship between the underlying disease and the related disease may be determined in accordance with instructions input by the user. The period for branching the related disease history information from the disease history information of the underlying disease may be specified based on instructions input by the user, or may be automatically specified based on the period in which medical information relating to the related disease was acquired. The period for branching the related disease history information from the disease history information of the underlying disease may be corrected by the user if the actual occurrence of the related disease is later than the specified period.

The disease history information display step may comprise a time series progression stop step for stopping the chronological progression of the disease history information relating to the specific disease during the period when treatment for the specific disease of the target patient has been completed. In this case, when treatment for the specific disease of the target patient ends (sometimes referred to as "discontinuation") due to cure of the disease or transfer of the patient to another hospital, the time at which the progression of the disease history information stops can be appropriately understood as the time when treatment was completed. Furthermore, by checking whether the chronological progression of the disease history information has stopped, it is also possible to determine whether treatment for the disease is ongoing. Thus, the efficiency and accuracy of patient treatment can be further improved.

Specific methods for stopping the chronological progression of the disease history information upon the end of treatment can be selected as appropriate. For example, the control unit may stop the chronological progression by ceasing extension of the line indicating the time series. Furthermore, when stopping the chronological progression of the related disease history information, the control unit may integrate the time series of the related disease history information into the time series of the underlying disease. In this case, medical professionals can appropriately identify that treatment of the related disease occurring in connection with the underlying disease has been completed by comparing it with the course of the underlying disease.

The disease history information display step may comprise a time-specific display step in which, for each time period indicated by the time series shown in the disease history information, one or more pieces of medical information corresponding to the respective time period are displayed on the display unit. In this case, medical professionals can appropriately identify multiple types of medical information corresponding to each time period while viewing the disease history information indicating the progression of the disease over time. Thus, the efficiency and accuracy of patient treatment can be further improved.

### (Third aspect)

A third aspect of the medical information processing program and medical information processing device of the present disclosure will be described. The medical information processing device exemplified in the present disclosure processes medical information relating to patients. The database is configured to store, for each patient, a plurality of types of medical information in association with the acquisition time of each piece of information. The control unit is configured to perform a patient identification, matrix display, and value transition display step. In the patient identification step, the control unit specifies a target patient whose medical information is to be processed. In the matrix display step, the control unit arranges at least part of the multiple types of medical information about the target patient along one axis by type, and along the other axis in chronological order of acquisition, and displays it as a matrix on the display unit. In the value transition information display step, the control unit displays value transition information showing changes in values over time for at least some types of medical information whose values may increase or decrease, together with the matrix display on the display unit.

According to the technology described in the present disclosure, multiple types of medical information are arranged by type along one axis and in chronological order along the other axis, and displayed in a matrix format on the display unit. Furthermore, value transition information showing changes in values for at least some of the medical information comprised in the matrix display is displayed together with the matrix display on the display unit. Therefore, medical professionals can appropriately identify each of the multiple types of medical information displayed in the matrix, as well as easily identify the value transitions of at least some of the medical information. As a result, necessary medical information is appropriately presented to medical professionals.

Various types of medical information (for example, at least some of medical images, examination results, findings, symptoms, prescriptions, treatment details, surgical details, etc.) may be comprised in the multiple types of medical information.

In the value transition information display step, the control unit may display, as value transition information, a graph that visualizes the transitions of at least some of the values of medical information, on the display unit. In this case, medical professionals can identify multiple types of medical information in a matrix display, while also easily identifying the transitions of the values of at least some of the medical information through graphs. Therefore, medical information can be more appropriately and easily understood.

In the value transition information display step, when the type of medical information is specified by the user on the matrix display, the control unit may display, as a value transition graph, the transitions of the values of the specified medical information on the display unit. In this case, medical professionals can identify multiple types of medical information in the matrix display, confirm the transitions of the values, and, by specifying the desired medical information as is on the matrix display, check the graph of the specified medical information. Therefore, the efficiency and accuracy of diagnosis can be further improved.

In the value transition information display step, when the period of the medical information is specified by the user on the matrix display, the control unit may display, as a value transition graph, the transitions of the values of the medical information within the specified period on the display unit. In this case, medical professionals can, by specifying the desired period on the matrix display showing multiple types of medical information, check the graph of the medical information for the specified period. Therefore, medical professionals can more easily identify the transitions of medical information within the necessary period.

In the value transition information display step, when a value specified by the user on the graph is designated, the control unit may display, on the matrix display, the value specified on the graph among the multiple values shown on the matrix display. In this case, medical professionals can easily confirm the value specified on the graph on the matrix display simply by designating the value on the graph. For example, it is possible to compare the value specified on the graph with other types of medical information acquired at the same time, using the matrix display, and perform diagnosis. Therefore, the efficiency and accuracy of diagnosis can be further improved.

In the value transition information display step, the control unit may display, on the matrix display as value transition information, the difference between at least some of the values of the medical information and the values of the same type of medical information acquired previously, as well as information indicating which side is closer to the reference value. In this case, medical professionals can identify multiple types of medical information using the matrix display, and also appropriately identify the value transitions of the medical information as difference information added to the matrix display. Therefore, medical information can be more appropriately and easily understood.

Specific methods for displaying difference information can be selected as appropriate. For example, when the value is higher than the previous value or the reference value, an upward arrow may be displayed; when the value is lower, a downward arrow may be displayed; and when the value is the same, a horizontal arrow may be displayed. If the difference is large, a larger number of arrows may be displayed. Also, if the difference is large, the size of the arrows may be increased. Additionally, the color assigned to the matrix display may be changed according to the difference from the previous value or the reference value, to display the difference information.

### (Fourth aspect)

A fourth aspect of the medical information processing program and medical information processing device of the present disclosure will be described. The medical information processing device exemplified in the present disclosure processes medical information relating to patients. The database is configured to store, for each patient, a plurality of types of medical information in association with the acquisition time of each piece of information. The control unit of the medical information processing device executes a patient identification step, a status information acquisition step, a display rule setting step, and a matrix display step. In the patient identification step, the control unit specifies a target patient whose medical information is to be processed. In the status information acquisition step, the control unit acquires information relating to the usage status of the medical information processing device and, at least, information relating to the status of the target patient. In the display rule setting step, the control unit sets, based on the status information acquired in the status information acquisition step, at least one display rule that defines rules for narrowing down the types of medical information to be displayed from among multiple types of medical information about the target patient, and rules for determining the display order of the multiple types of medical information. In the matrix display step, the control unit executes, for the multiple types of medical information relating to the target patient, at least one of a type narrowing process and a display order determination process in accordance with the display rule for the disease set in the display rule setting step, then arranges the medical information in display order along one axis, and arranges them along the other axis in chronological order of acquisition, and displays them on the display unit in a matrix format.

According to the technology described in the present disclosure, based on at least one piece of status information relating to the usage status of the medical information processing device and/or the status of the target patient, multiple display rules for medical information about the target patient are set. For multiple types of medical information, at least one of narrowing down the types of medical information to be displayed on the display unit, and determining the display order (hereinafter referred to as "display information adjustment processing") is performed in accordance with the display rules set for the disease. Multiple types of medical information that have undergone display information adjustment processing are displayed in a matrix format on a display unit according to the display order and chronological order (hereinafter referred to as a "matrix display"). Accordingly, among the many types and large amounts of medical information, the medical information (some or all) defined by the display rules according to the usage status of the medical information processing device and/or the status of the target patient is listed in a matrix format in an appropriate display order. As a result, necessary medical information is appropriately presented to medical professionals.

Various types of medical information (for example, at least some of medical images, examination results, findings, symptoms, prescriptions, treatment details, surgical details, etc.) may be comprised in the multiple types of medical information.

In the status information acquisition step, the control unit may acquire occupation information that can specify the occupation of the user using the medical information processing device, as status information. In the display rule setting step, the control unit may set display rules corresponding to the occupation of the user of the medical information processing device specified by the occupation information. The occupation of the user using the medical information processing device (for example, at least one of physician, nurse, pharmacist, radiological technologist, physical therapist, occupational therapist, clinical psychologist, certified orthoptist/CO, nutritionist, etc.) may differ, and the importance of each type of medical information may vary depending on the occupation. Therefore, by following display rules corresponding to the occupation, multiple types of medical information can be displayed in a matrix format, making it easier to appropriately present necessary medical information to each medical professional.

The specific form of occupation information that can specify the occupation can be selected as appropriate. For example, when the user logs into the medical information processing device, the occupation of the user may be comprised in the login information (such as the user's ID, username, etc.). Additionally, the login information may be associated with the occupation of the user. In this case, the control unit may acquire the occupation information that can specify the occupation of the user from the login information. Alternatively, the user may input information indicating their own occupation into the medical information processing device. In this case, the information input by the user may be comprised as occupation information.

In the status information acquisition step, the control unit may acquire information specifying the location where the medical information processing device is used, as status information. In the display rule setting step, the control unit may set display rules corresponding to the specified location based on the usage location information. The medical information processing device may be used in various places (for example, in various examination rooms, consultation rooms, operating rooms, reception areas, or even at the patient's home or other locations outside the medical facility). The importance of each type of medical information may differ depending on the location where the medical information processing device is used. Therefore, by following display rules corresponding to the usage location of the medical information processing device, multiple types of medical information can be displayed in a matrix format. This makes it easier to appropriately present necessary medical information to healthcare workers engaged in medical care at each location.

The specific form of usage location information that can specify the location can be selected as appropriate. For example, when the medical information processing device is used at a specified location, usage location information indicating the usage location of the medical information processing device may be set for each medical information processing device. In this case, the control unit may acquire the usage location information set in the medical information processing device from storage devices or the like. Additionally, when the medical information processing device can be moved and used at multiple locations, the user may input information indicating the usage location into the medical information processing device via an operation unit or similar means. In this case, the information input via the operation unit or similar means may be used as usage location information. Furthermore, when the medical information processing device can acquire location information, the control unit may acquire the acquired location information as usage location information.

In the status information acquisition step, the control unit may acquire, as status information, difference information indicating the difference between at least some types of medical information about the target patient and the latest medical information or reference medical information of the same type for a comparison subject. In this case, the control unit may acquire, as status information, difference information showing the difference between the latest medical information about the target patient and the reference medical information for a comparison subject, which may be the latest information for a healthy individual or a previous examination result for the same patient. In the display rule setting step, the control unit may set display rules according to the difference information. For example, if the reference medical information is for a healthy individual, the greater the difference between the latest information for the target patient and the reference information, the higher the likelihood that the target patient has a disease. Also, if the reference medical information is a previous examination result for the same patient, the greater the difference between the latest information and the previous information, the higher the likelihood that the condition of the target patient has changed (either worsened or improved). Therefore, by setting display rules for multiple types of medical information based on the difference information between the latest information and the reference information, the control unit can make it easier for users to appropriately identify the condition of the target patient.

In the display rule setting step, the control unit may set display rules so that at least some of the multiple types of medical information about the target patient, with the largest differences indicated by the difference information, are displayed in the upper row of multiple horizontal rows, or in the leftmost column of multiple vertical columns. In this case, the medical information for which the difference from the reference information is greater is displayed in order from the top or left. Therefore, users can easily identify the types of medical information that have the largest differences from the reference information.

When setting display rules for multiple types of medical information according to the size of the difference, the specific format can be selected as appropriate. For example, the control unit may acquire, as difference information, the deviation of the latest information for each of multiple types of medical information from the comparison subject information. The control unit may set display rules so that, the larger the acquired deviation for a type of medical information, the higher the type is displayed in the horizontal rows or the further left in the vertical columns among the multiple rows or columns. In this case, multiple types of medical information with mutually different deviations are displayed in the display unit in an appropriate order according to the deviation.

However, it is also possible to change the method of setting display rules based on the difference information. For example, in the display rule setting step, among the multiple types of medical information for the target patient, the control unit may set display rules so that one or more types of medical information for which the difference indicated by the difference information falls below a threshold are displayed in the upper row of multiple horizontal rows or in the leftmost column of multiple vertical columns, rather than those for which the difference exceeds the threshold. In this case as well, the user can check the upper rows or leftmost columns of the matrix display to easily identify the medical information for which the difference from the comparison subject information exceeds the threshold. When using a threshold, the difference information may be the deviation described above, or it may be the difference in value between the latest information and the comparison subject information. The threshold may be set for each type of medical information. Additionally, the control unit may set the display style (for example, the color of the frame, the color of the text, the presence or absence of a mark, etc.) for the medical information for which the difference indicated by the difference information exceeds the threshold, and set a different display style for the medical information for which the difference falls below the threshold. In this case, the user can easily identify the medical information for which the difference from the comparison subject information exceeds the threshold, based on the display style of the medical information.

In the status information acquisition step, the control unit may acquire, for at least one of multiple types of medical information about the target patient, the latest medical information that has been most recently obtained, and, as comparison subject information, the same type of medical information obtained for the same patient at one point prior to the latest information, and may acquire, as status information, difference information indicating the difference between the comparison subject information and the latest information. As described above, when the comparison subject information is medical information obtained at the previous examination for the same patient (hereinafter referred to as "previous information"), the greater the difference between the latest information and the previous information, the higher the likelihood that the condition of the target patient has changed (worsened or improved). Therefore, by setting display rules for multiple types of medical information based on the difference information between the latest information and the previous information, the control unit can make it easier for users to appropriately identify changes in the condition of the target patient.

For example, it is also possible to change the specific form of the comparison subject information. For example, as described above, medical information of a healthy subject without disease (for example, a normal value, average value, reference value, or permissible range, etc.; any of these with a small range may be used) can be used as the comparison subject information. Alternatively, without limiting the comparison subject information to the medical information obtained immediately prior to the latest information, medical information of the same type obtained for the same subject on a specific day may be used as the comparison subject information. In this case as well, the condition of the patient can be more appropriately identified by the user. Furthermore, the control unit may comprise, as the comparison subject information, the latest medical information as well as multiple pieces of medical information (for example, three or more times) obtained for the same subject, and may acquire, as status information, the rate of change (for example, the trend of values, etc.) of the same type of medical information for the same subject, and handle it as difference information. The control unit may also set display rules according to the acquired difference information. For example, the control unit may display, in the upper row of multiple horizontal rows or in the leftmost column of multiple vertical columns, the medical information for which the rate of change between the latest information and the previous information is greater than or equal to the rate of change before the previous information (for example, the average rate of change before the previous information, etc.). Alternatively, the control unit may set display rules so that the greater the difference from the rate of change between the latest information and the previous information (for example, the average rate of change before the previous information, etc.), the higher the medical information is displayed in the upper row of multiple horizontal rows or the further left in the leftmost column of multiple vertical columns. In this case, the medical information for which the rate of change becomes greater can be more easily identified by the user.

In the status information acquisition step, the control unit may acquire, as status information, scheduled medical information to be obtained for the target patient on the day, and among them, information that has not yet been obtained as of that day, referred to as not obtained information. In the display rule setting step, the control unit may set display rules such that, based on the not obtained information, items of medical information that have not yet been obtained as of that day are displayed in the upper row among multiple horizontal rows, or in the leftmost column among multiple vertical columns, compared to other medical information items. In this case, the user can check the upper row or the leftmost column to easily identify, among the scheduled medical information to be obtained for the target patient on the day, which information has not yet been obtained.

Additionally, the control unit may set the display style (for example, the color of the frame, the color of the text, the presence or absence of a mark, etc.) for items of medical information that have not yet been obtained as of that day, to a display style different from that of other medical information items. In this case, the user can easily identify, based on the display style of the medical information, which scheduled medical information to be obtained for the target patient on the day has not yet been obtained.

Furthermore, in the status information acquisition step, the control unit may acquire, as status information, scheduled medical information to be obtained for the target patient on the day (including both information already obtained and information not yet obtained as of that day). In the display rule setting step, the control unit may set display rules such that, for items of scheduled medical information to be obtained for the target patient on the day, those items are displayed in the upper row among multiple horizontal rows, or in the leftmost column among multiple vertical columns, compared to other medical information items. In this case, the user can check the upper row or the leftmost column to easily identify, among the scheduled medical information to be obtained for the target patient on the day, as well as the medical information already obtained on that day, the relevant information.

Among the multiple display rules, there may be patient display rules established for allowing the patient to view multiple types of medical information. The control unit, in accordance with instructions input by the user, may execute display information adjustment processing based on the patient display rules if such rules are set. After that, multiple types of medical information may be displayed in a matrix format according to the patient display rules. It is also possible to display multiple types of medical information in a matrix format based on the patient display rules. For each type of medical information, the necessity for the patient to view the information may differ. Therefore, by displaying multiple types of medical information in a matrix format based on the patient display rules, it becomes easier to appropriately present medical information to the patient.

### (Fifth aspect)

A fifth aspect of the medical information processing program and medical information processing device of the present disclosure will be described. The medical information processing device exemplified in the present disclosure processes medical information relating to patients. The database is configured to store, for each patient, a plurality of types of medical information in association with the acquisition time of each piece of information. The control unit of the medical information processing device executes a patient identification step, a matrix display step, a column/row selection reception step, and a column/row information display step. In the patient identification step, the control unit specifies a target patient whose medical information is to be processed. In the matrix display step, the control unit arranges multiple types of medical information about the target patient in vertical columns and horizontal rows by type, and arranges information by acquisition time in the other direction, displaying them as a matrix on the display unit. In the column/row selection reception step, the control unit receives instructions from the user to select at least one column/row among one or more vertical columns and one or more horizontal rows in the matrix display. In the column/row selection reception step, among the vertical columns and horizontal rows in the matrix display, if the column/row selected in the column/row selection reception step is a vertical column, the control unit extracts, from the medical information of the target patient stored in the database, the medical information of the type or acquisition time displayed in the selected vertical column and displays it on the display unit. If the column/row selected in the column/row selection reception step is a horizontal row, the control unit extracts, from the medical information of the target patient stored in the database, the medical information of the type or acquisition time displayed in the selected horizontal row and displays it on the display unit.

According to the technology described in the present disclosure, multiple types of medical information are displayed in a matrix format according to each type and the time of acquisition. Therefore, a large number and variety of types of medical information can be appropriately listed and displayed. Furthermore, when a column/row (vertical column or horizontal row) in the matrix display is selected by the user, the type or the medical information at the acquisition time displayed in the selected column/row is extracted and displayed. Therefore, by simply selecting the column/row (vertical or horizontal) in the matrix display for which the user wants to check the information, it is possible to easily check the medical information of the selected column/row. As a result, necessary medical information is appropriately presented to medical professionals.

Various types of medical information (for example, at least some of medical images, examination results, findings, symptoms, prescriptions, treatment details, surgical details, etc.) may be comprised in the multiple types of medical information.

In the matrix display step, the control unit may, for at least some of the multiple types of medical information about the target patient, display, on the display unit, simplified information in which part of the information has been extracted or reduced, or simple information in which image information has been reduced. In this case, the user can appropriately identify multiple types of medical information based on the simplified information, which is displayed in a matrix format after extraction or reduction of the information. Furthermore, in the column/row selection reception step, the control unit may, for at least some of the multiple types of medical information about the target patient, display, on the display unit, more detailed or expanded information than the simplified information displayed in the matrix display. In this case, by selecting the column/row for which the user wants to check the information, the user can identify the medical information in the selected column/row in more detail than the simplified information displayed in the matrix display. Therefore, necessary medical information is presented to healthcare workers in a more appropriate manner.

In the column/row selection reception step, the control unit may, from the matrix display shown in the matrix display step, transfer the medical information displayed in the selected column/row to the column information display unit in the column/row selection reception step. In this case, by the transition of the display style, the user can more easily identify that the information shown on the display screen has transitioned to the medical information of the selected column/row. Furthermore, the control unit may display the medical information of the selected column/row in the column/row information display unit using a display method different from that in the matrix display unit. In this case, necessary medical information is more easily identified.

The control unit may further execute a cell selection receiving step and a cell information display step. In the cell selection receiving step, the control unit receives an instruction to select at least one of the multiple cells arranged in a grid in the matrix display. In the cell information display step, the control unit displays, as more detailed or expanded information, the medical information of the cell selected in the cell selection receiving step, compared to the simplified information shown in the matrix display. In this case, the user can select the cell for which they wish to confirm information, and thus, it is possible to appropriately identify the detailed medical information of the selected cell, beyond the simplified information shown in the matrix display. Therefore, necessary medical information is presented to healthcare workers in a more appropriate manner.

In the column/row selection reception step, the control unit may receive an instruction to select at least two vertical columns among multiple vertical columns in the matrix display, or at least two horizontal rows among multiple horizontal rows. If two or more columns/rows are selected in the column/row selection reception step, the control unit may extract, from the database, the medical information for the types or acquisition times displayed in the selected two or more vertical columns and/or horizontal rows, and display it on the display unit. In this case, the user can easily identify the medical information of multiple columns/rows by simply selecting multiple columns/rows in the matrix display. Thus, necessary medical information is appropriately presented to healthcare professionals.

In the column/row selection reception step, when multiple types of medical information displayed by the column/row selection reception step comprise both image information and numerical information, the control unit may, in accordance with instructions input by the user, perform a re-display step in which only either the image information or the numerical information is extracted from the multiple types of medical information being displayed and displayed on the display unit. In this case, the user can further identify in detail only either the image information or the numerical information from among the multiple types of medical information corresponding to the selected column/row. Thus, necessary medical information is appropriately presented to healthcare professionals.

In the database, it is possible to store, among the tissues existing on the left and right sides of the human body (for example, eyes, ears, nasal cavity, hands, feet, etc.), medical information for each tissue on the left and right sides for each patient. In the column/row selection reception step, when multiple types of medical information displayed comprise both medical information for the left tissue and medical information for the right tissue, the control unit may, in accordance with instructions input by the user, perform a left-right extraction display step in which only either the medical information for the left tissue or the medical information for the right tissue is extracted from the multiple types of medical information and displayed on the display unit. In this case, the user can further identify in detail only either the medical information for the left tissue or the medical information for the right tissue from among the multiple types of medical information corresponding to the selected column/row. As a result, necessary medical information is appropriately presented to medical professionals.

The control unit may further execute an image selection receiving step, an enlarged display step, and an image processing step. In the image selection receiving step, the control unit receives an instruction to select at least one piece of image information from among multiple pieces of medical information displayed in the matrix display step or in a separate information display step. In the enlarged display step, the control unit enlarges and displays, on the display unit, the image information selected in the image selection receiving step, compared to the image displayed in the matrix display step or the separate information display step. In the image processing step, the control unit is configured to perform processing in accordance with the instruction input by the user for the image information enlarged and displayed in the enlarged display step. In this case, the user can select the desired image information from among the multiple pieces of medical information displayed in the matrix display step or the separate information display step, enlarge and display the selected image information, and perform various processing on the enlarged image information. Thus, necessary medical information is appropriately presented to healthcare professionals.

In the image processing step, the control unit may appropriately select processing that can be performed on the image information. For example, the processing may comprise enlargement processing, reduction processing, extraction processing, drawing processing on the image, measurement of various values, synthesis processing (for example, synthesis of multiple images such as parameter synthesis), transfer processing to other devices, or the like.

Among the techniques exemplified in the first aspect through the fifth aspect, it is also possible to combine multiple techniques that are exemplified in different aspects.

### [Brief Description of Drawings]

[FIG. 1] Block diagram showing the schematic configuration of the medical information processing system 1.
[FIG. 2] Conceptual diagram schematically illustrating an example of medical information stored in the database.
[FIG. 3] Flowchart of medical information processing executed by the medical information processing device 10 according to the first embodiment.
[FIG. 4] Diagram showing an example of a screen in which medical information of a target patient is displayed in a matrix format according to a default display rule.
[FIG. 5] Diagram showing an example of a screen in which the matrix display of medical information is changed according to a display rule corresponding to glaucoma, from the default display state shown in FIG. 4.
[FIG. 6] Flowchart of medical information processing executed by the medical information processing device 10 according to the second embodiment.
[FIG. 7] Diagram showing an example of the default matrix display of medical information and the display of disease history information in the second embodiment.
[FIG. 8] Diagram showing an example of a screen in which the matrix display of medical information is changed according to a display rule corresponding to concurrent cataract, from the default display state shown in FIG. 7.
[FIG. 9] Diagram showing an example of a screen in which the time-specific display section 43 is displayed in the second embodiment.
[FIG. 10] Flowchart of medical information processing executed by the medical information processing device 10 according to the third embodiment.
[FIG. 11] Diagram showing an example of a matrix display of medical information and display of value transition information in the third embodiment.
[FIG. 12] Diagram showing an example of a screen when the display period range of the graph is specified on the matrix display section 30, in the display state shown in FIG. 11.
[FIG. 13] Diagram showing an example of a screen when a specific value in the graph is specified, in the display state shown in FIG. 11.
[FIG. 14] Flowchart of medical information processing executed by the medical information processing device 10 according to the fourth embodiment.
[FIG. 15] Diagram showing an example of a screen in which medical information of a target patient is displayed in a matrix format according to a display rule corresponding to the occupation.
[FIG. 16] Diagram showing an example of a screen in which medical information of a target patient is displayed in a matrix format according to a display rule corresponding to the difference between the latest information and the comparison information.
[FIG. 17] Diagram showing an example of a screen in which medical information of a target patient is displayed in a matrix format according to a display rule corresponding to the schedule for the day.
[FIG. 18] Flowchart of medical information processing executed by the medical information processing device 10 according to the fifth embodiment.
[FIG. 19] Diagram showing an example of a matrix display of medical information in the fifth embodiment.
[FIG. 20] Diagram showing an example of the column information display section 70 displayed when a specific column of the matrix display section 30 shown in FIG. 19 is selected.
[FIG. 21] Diagram showing an example of the drawing information processing screen 80.

### [DESCRIPTION OF EMBODIMENTS]

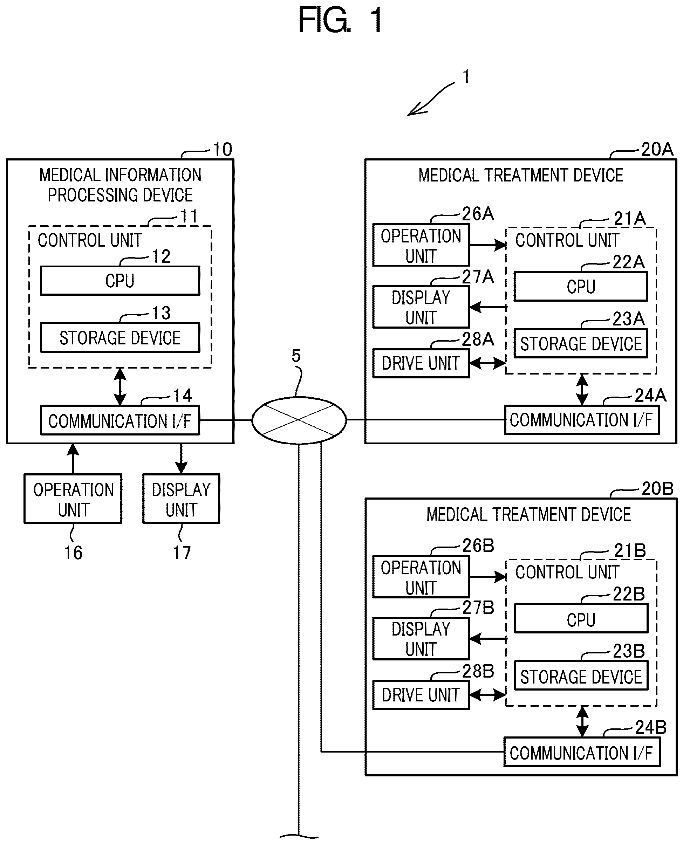

Hereinafter, a typical embodiment in the present disclosure will be described with reference to the drawings. First, with reference to FIG. 1, an overview of an example of the system configuration of the medical information processing system 1 in the present embodiment will be described. In the present embodiment, a medical information processing system 1 that processes ophthalmological medical information will be exemplified and described. However, the techniques exemplified in the present disclosure can also be applied to cases where medical information in medical fields other than ophthalmology is processed. The medical information processing system 1 of the present embodiment comprises a medical information processing device 10 and medical treatment devices 20 (20A, 20B).

The medical information processing device 10 processes various types of medical information. In the present embodiment, as the medical information processing device 10, a personal computer (hereinafter referred to as "PC") installed in a medical facility is used. However, the medical information processing device 10 is not limited to a PC. For example, a smartphone, tablet terminal, or other mobile terminal may also be used as the medical information processing device 10. A server may be used separately from or together with a PC as the medical information processing device 10. In this case, the server may be, for example, a server of a manufacturer that provides cloud services (a so-called cloud server), or a server other than a cloud server (for example, a server of a manufacturer that provides a medical information processing program). Multiple devices may collaborate to function as the medical information processing device 10.

The medical information processing device 10 comprises a control unit 11, which is configured to perform various types of control processing, and a communication interface (I/F) 14. The control unit 11 comprises a CPU 12, which is a controller responsible for control, and a storage device 13 capable of storing programs and data. The communication interface 14 connects the medical information processing device 10 to external devices (for example, medical treatment devices 20 and the like) via the network 5. The medical information processing device 10 is also connected to the operation unit 16 and the display unit 17. The operation unit 16 is operated by the user (in the present embodiment, medical professionals such as physicians, nurses, and technicians) to input various instructions into the medical information processing device 10. The display unit 17 displays various images.

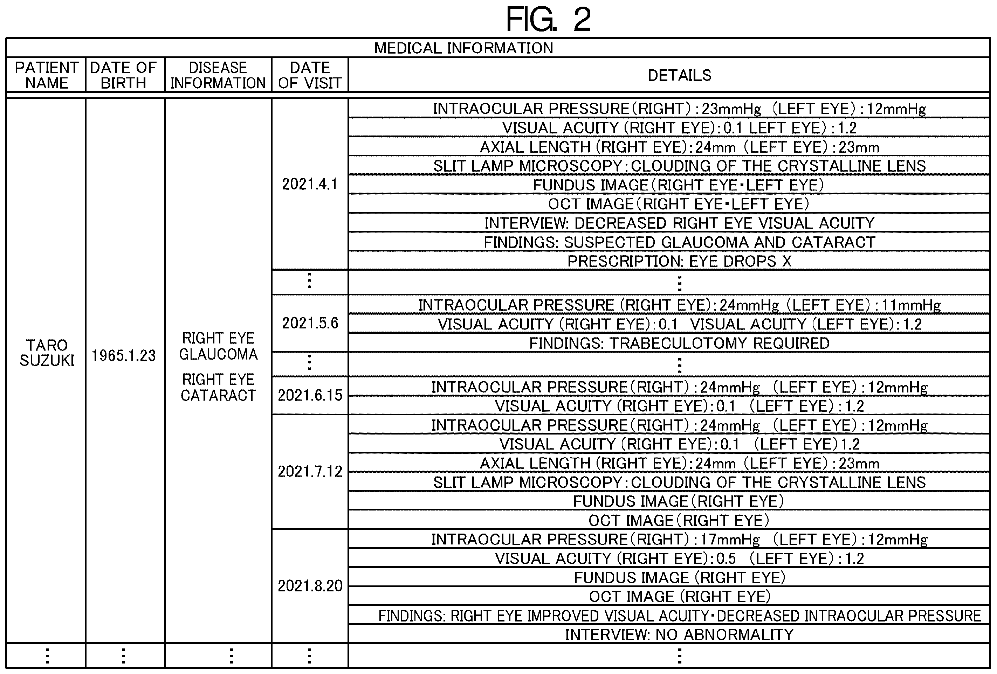

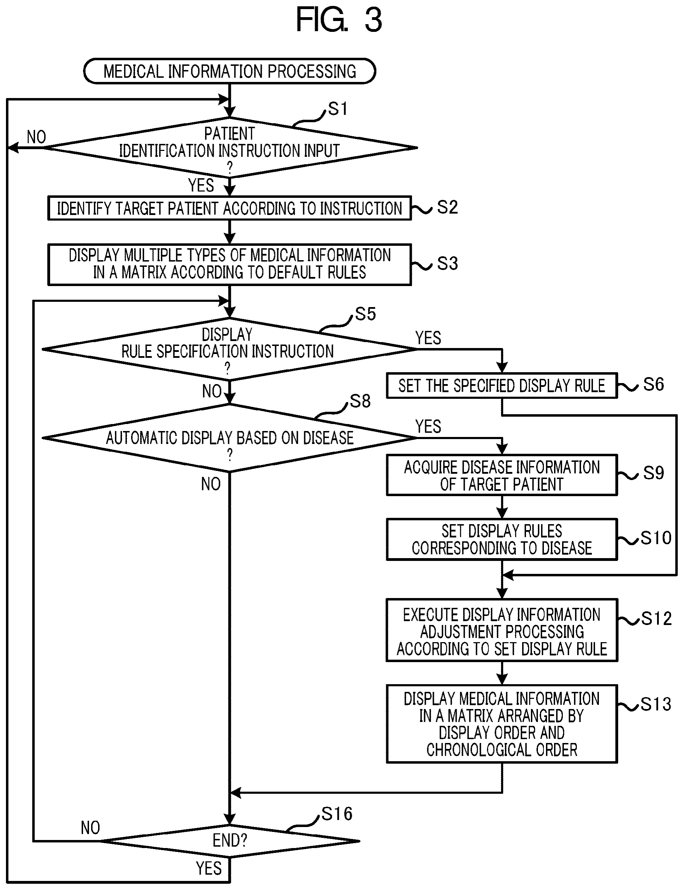

The storage device 13 stores a medical information processing program for executing at least part of the medical information processing described later (see FIGS. 3, 6, and 10). In addition, the storage device 13 stores, for each patient, multiple types of medical information, each associated with the time of acquisition of the respective information. Details of the medical information will be described later with reference to FIG. 2.

In this embodiment, as an example, a case is illustrated in which medical information is stored in the storage device 13 built into the medical information processing device 10. However, the storage device in which the medical information is stored may be provided outside the medical information processing device 10. For example, medical information may be stored on a server (including a cloud server) or on an external hard disk. In this case, the medical information processing device 10 and the storage device may be connected by wireless communication (including networks such as the Internet) or by wired communication.

The medical treatment device 20 (20A, 20B) is configured to perform at least one of various medical treatments such as examination, imaging, treatment, or surgery on a patient (in the present embodiment, the eye to be examined of the patient). As the medical treatment device 20, for example, at least one of an OCT device, a scanning laser ophthalmoscope (SLO), a fundus camera, a corneal endothelial cell imaging device, an axial length measuring device, a refractometer, or a tonometer may be employed. The medical treatment device 20 comprises a control unit 21 (21A, 21B) that is configured to perform various control processes, and a communication interface 24 (24A, 24B). The control unit 21 comprises a CPU 22 (22A, 22B), which is a controller responsible for control, and a storage device 23 (23A, 23B) capable of storing programs and data. In addition, the medical treatment device 20 comprises an operation unit 26 (26A, 26B), a display unit 27 (27A, 27B), and a drive unit 28 (28A, 28B). The drive unit 28 comprises various components necessary for the medical treatment device 20 to perform treatment on the patient.

### <Medical information>

With reference to FIG. 2, an example of medical information stored in the database in the present embodiment will be described. As illustrated in FIG. 2, in the storage device 13 (an example of a database), for each patient, multiple types of medical information are stored, each associated with the time of acquisition of the respective information (as an example, in the present embodiment, "date of medical examination"). The medical information in the present embodiment refers to information regarding at least one of examinations, imaging, and medical consultations performed on a patient. As an example, in the present embodiment, the following are processed as medical information: image data of the eye to be examined captured by the medical treatment device (imaging device) 20 (for example, at least one type of image data such as OCT images, SLO images, fundus camera images, or corneal endothelial cell images); examination data indicating the results of examinations of the eye to be examined performed by the medical treatment device (examination device) 20 (for example, at least one type of examination data such as axial length, refractive power, or intraocular pressure); data indicating the content of treatment or surgery of the eye to be examined performed by the medical treatment device (treatment device or surgical device) 20; and information such as the results of medical consultations for the patient (including at least one of findings, interview results, symptoms claimed by the patient, prescriptions, treatment details, surgical details, examination results, and diagnosis results) created by operation of the operation unit 16 by the user.

In the example shown in FIG. 2, multiple types of medical information (for example, measurement results of intraocular pressure, measurement results of visual acuity, measurement results of axial length, examination results by slit lamp microscopy, fundus images, OCT images, interview results, findings, prescriptions, and so on) are stored for each patient, associated with the date of medical examination. In addition, in the example shown in FIG. 2, as information for each patient, not only the patient's name and date of birth, but also information regarding the patient's disease (disease information) is stored in the storage device 13. Furthermore, in the present embodiment, among the multiple types of medical information, there are types of medical information whose values may increase or decrease (in the example shown in FIG. 2, measurement results of intraocular pressure, measurement results of visual acuity, and measurement results of axial length are comprised).

### (First embodiment)

With reference to FIGS. 3 to 5, the medical information processing executed by the medical information processing device 10 of the first embodiment will be described. In the medical information processing of the first embodiment (see FIG. 3), for multiple types of medical information, at least one of a narrowing process for the types of medical information to be displayed on the display unit, and a determination process for the display order (hereinafter referred to as "display information adjustment processing") is executed according to the set display rule. The multiple types of medical information that have undergone display information adjustment processing are displayed in a matrix format on the display unit 17 according to each of the display order and chronological order. The medical information processing is executed by the CPU 12 of the medical information processing device 10 in accordance with the medical information processing program stored in the storage device 13.

As shown in FIG. 3, when the CPU 12 starts processing medical information, it determines whether an instruction specifying the patient (target patient) whose medical information is to be processed has been input (S1). For example, when the user operates the operation unit 16, if an instruction specifying the target patient (for example, inputting the patient's name or patient ID) is input into the medical information processing device 10, the patient is specified. Additionally, information for identifying the patient (for example, a barcode, etc.) assigned to each patient may be used. In this case, the CPU 12 may receive an instruction to specify the target patient by inputting the identifier read by the identifier reader. If the instruction specifying the target patient has not been input (S1: NO), the process of S1 is repeated and the state is maintained.

When an instruction to specify the target patient is input (S1: YES), the CPU 12 specifies the patient to be processed for medical information according to the input instruction (S2). In the following, as an example, a case in which Taro Suzuki, shown in FIG. 2, is specified as the target patient will be described.

Next, the CPU 12, following the default display rule, determines the display order of multiple types of medical information related to the target patient (for example, in the present embodiment, S3 is the display order of all medical information for the target patient), and displays the multiple types of medical information in a matrix format on the display unit 17 according to the determined display order (S3).

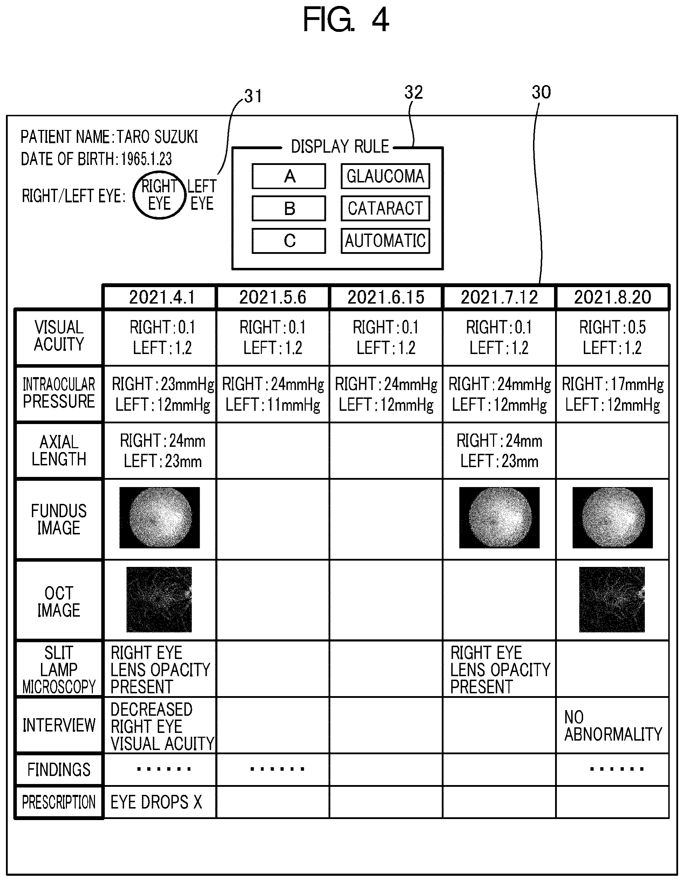

FIG. 4 is a diagram showing an example of a screen in which the medical information of a target patient is displayed in a matrix format according to the default display rule. As shown in FIG. 4, in the matrix display 30, multiple types of medical information are arranged and displayed in one direction (vertical in FIG. 4) according to the display rule for types (from top to bottom in FIG. 4), and in the other direction (horizontal in FIG. 4), each type of medical information is displayed in chronological order (from left to right in FIG. 4). Therefore, by referring to the matrix display 30, medical professionals can appropriately identify multiple types of medical information related to the target patient in chronological order.

In the upper left part of the screen shown in FIG. 4, the name and date of birth of the target patient are displayed, along with a selection section 31 for selecting which eye (right eye or left eye) to display medical information for. The user operates the operation unit 16 to select either "right eye" or "left eye" in the eye selection section 31, thereby switching the eye for which medical information is displayed in the matrix display section 30 between the right and left eyes.

As shown in the upper central part of the screen in FIG. 4, a display rule selection section 32 is displayed. In the first embodiment, the medical information processing device 10 is configured such that a plurality of display rules for displaying multiple types of medical information in a matrix format can be registered. Each display rule specifies at least one of a rule for narrowing down the types of medical information to be displayed in a matrix format from among the multiple types of medical information for the target patient, and a rule for determining the display order of the multiple types of medical information. In other words, for each display rule, the narrowing rule for the types of medical information to be displayed in the matrix format, and the rule for determining the display order of the multiple types of medical information to be displayed in the matrix format, are set. The user operates the operation unit 16 to select (specify) one of the plurality of display rules, and the medical information is displayed on the screen 17 in accordance with the selected display rule.

The CPU 12 of the medical information processing device 10 can execute display rule generation processing. In the display rule generation processing, the CPU 12, via the operation unit 16, generates a display rule according to an instruction input by the user, and stores it in the database (in the present embodiment, in the storage device 13). Therefore, medical professionals can generate and register a display rule suitable for their own use by appropriately combining multiple types of medical information and specifying the display format, such as matrix format, as needed. Thus, necessary medical information can be presented more appropriately to medical professionals. For example, as shown in FIG. 4, three display rules [A],[B], and[C] are registered, and a display rule selection section 32 is displayed, which allows the user to select each display rule by pressing the corresponding button.

In addition, in the first embodiment, among the multiple display rules, there are comprised multiple display rules that are defined for each of a plurality of diseases. When treating patients, the importance of each type of medical information often varies depending on the patient's disease. Accordingly, when medical information is displayed in a matrix format on the display unit in accordance with display rules that correspond to the disease of the target patient, it becomes easier for medical professionals to appropriately treat the disease based on the displayed medical information. As shown in FIG. 4, a display rule corresponding to glaucoma for internal medicine and a display rule corresponding to cataract for ophthalmology can be set, and each display rule is displayed as a button in the display rule selection section 32. The display rule corresponding to the disease may be a predetermined rule, or it may be defined according to instructions input by the user.

In the first embodiment, the user can, based on the display rule corresponding to the disease indicated by the disease information of the target patient, automatically display multiple types of medical information in a matrix format. Therefore, the efficiency of medical care by medical professionals can be further improved. As shown in FIG. 4, for example, the user can operate the operation unit 16 to specify "automatic" in the display rule selection section 32, so that the medical information processing device 10 can automatically execute the display of medical information in accordance with the disease of the target patient.

Returning to the explanation in FIG. 3. The CPU 12 determines whether at least one of a plurality of display rules has been specified (S5). If not specified (S5: NO), the CPU 12 determines whether automatic display according to the disease of the subject patient has been selected (S8). If automatic display has not been selected (S8: NO), it is determined whether there is an instruction to execute the display processing of medical information for the subject patient (S16). If there is no instruction to execute the display processing (S16: NO), the process returns to S5, and the processing from S5 to S13 is repeatedly executed. If an instruction to terminate the display processing is input (S16: YES), the process returns to S1.

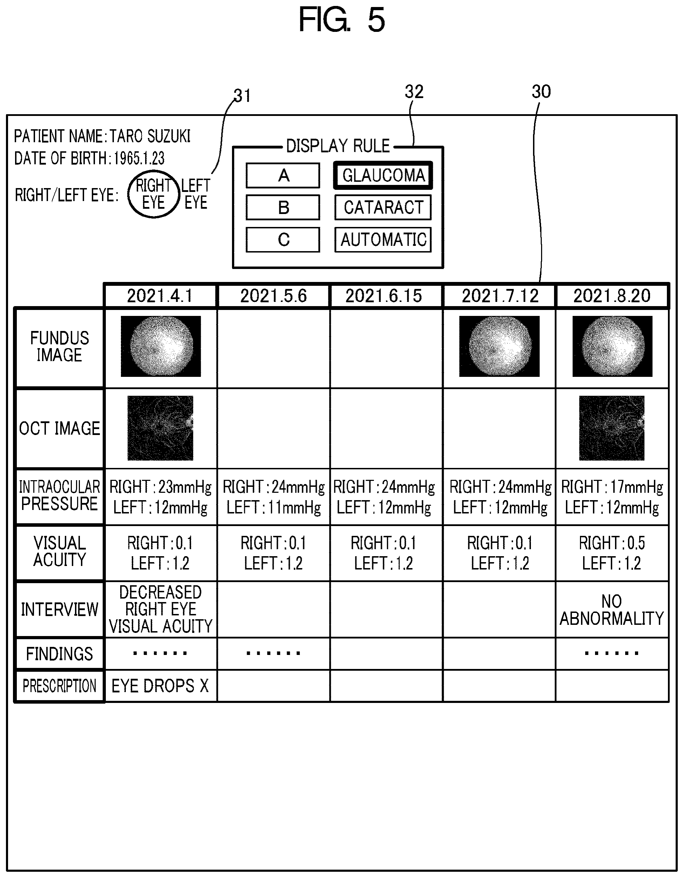

When at least one of the multiple display rules indicated in the display rule selection section 32 is specified (selected) (S5: YES), the CPU 12 sets the specified display rule as the display rule for performing display information adjustment processing (S6). Next, the CPU 12, in accordance with the set display rule, executes display information adjustment processing (that is, at least one of a narrowing process for the types of multiple kinds of medical information for the target patient, and a process for determining the display order) (S12). The CPU 12, in accordance with the result of the display information adjustment processing, displays multiple types of medical information in a matrix format (S13). In other words, the CPU 13 arranges multiple types of medical information in one of the vertical or horizontal rows according to the display order, and arranges and displays each type of medical information in the other column in chronological order. As a result, as shown in FIG. 5, among the many types and amounts of medical information, the medical information (some or all of the medical information) specified by the display rule is listed and displayed in a matrix format in the appropriate display order. As a result, necessary medical information is appropriately presented to medical professionals.

FIG.5 is a diagram showing an example of a screen in which the matrix display of medical information is changed, from the default display state shown in FIG. 4, according to the display rule corresponding to glaucoma. In the example shown in FIG. 5, among the multiple types of medical information, several types of medical information that are highly important for the diagnosis and treatment of glaucoma are narrowed down, and these multiple types of medical information are arranged in order of importance for glaucoma diagnosis and treatment, from top to bottom, and displayed in the matrix display section 30.

Also, in the case where automatic display corresponding to the patient's disease is selected (S8: YES), the CPU 12 acquires disease information related to the subject patient (S9). As an example, in the present embodiment, a part of the patient's medical information comprises disease information (see FIG. 2). In S9, the CPU 12 refers to the disease information comprised in the medical information related to the subject patient. Furthermore, in the present embodiment, a mechanism or program for outputting an automatic determination result of the patient's disease using a mathematical model is provided. The mathematical model is pre-trained so that, when the medical information of the target patient is input, it outputs an automatic determination result of the disease of the target patient. The CPU 12 can also obtain, as disease information of the target patient, the automatic determination result output by the mathematical model by inputting the medical information of the target patient (for example, image data, etc.) into the mathematical model.

CPU 12 automatically sets a display rule corresponding to the disease indicated by the disease information of the target patient obtained in S9 (S10). CPU 12 executes display information adjustment processing according to the set display rule (S12). The CPU 12, in accordance with the result of the display information adjustment processing, displays multiple types of medical information in a matrix format (S13). As a result, multiple types of medical information are automatically displayed in a matrix format according to the display rule corresponding to the disease information of the target patient.

### (Second embodiment)

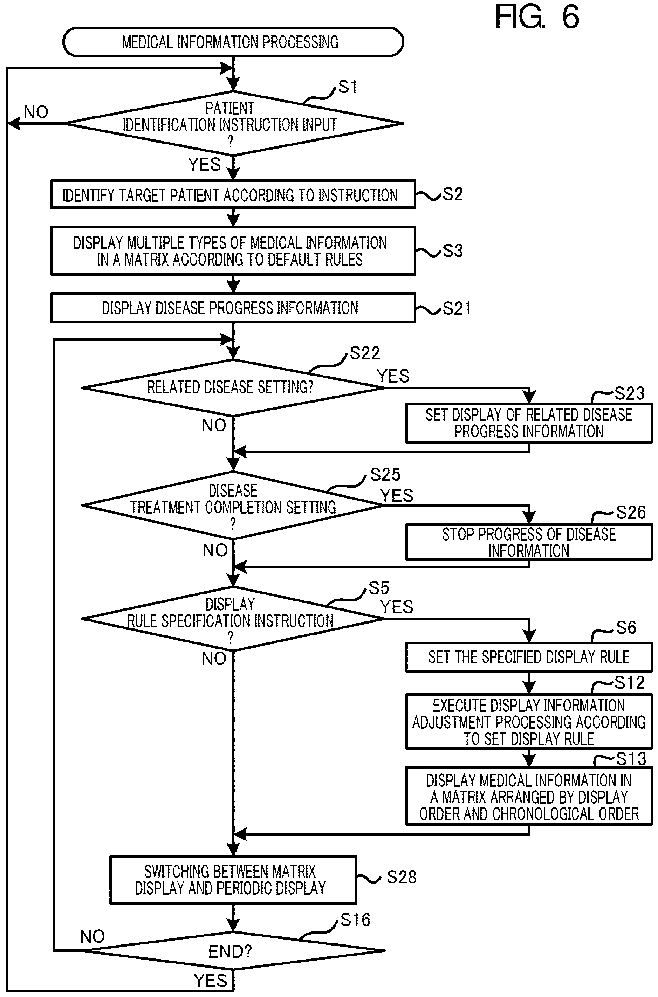

With reference to FIGS. 6 to 9, the medical information processing executed by the medical information processing device 10 of the second embodiment will be described. In the medical information processing of the second embodiment (see FIG. 6), display information adjustment processing for multiple types of medical information is executed based on at least one of a plurality of display rules defined for each of a plurality of diseases, and the information is displayed in a matrix format. In addition, in the second embodiment, disease progress information (which may also be referred to as a "timeline" in this embodiment) is displayed, showing the course of a specific disease of the target patient in chronological order. The medical information processing is executed by the CPU 12 of the medical information processing device 10 in accordance with the medical information processing program stored in the storage device 13. It is also possible for at least some of the steps in the medical information processing of the second embodiment to employ the same processing as the steps in the medical information processing of the first embodiment. Accordingly, for steps that can employ the same processing as the steps in the first embodiment, the same step numbers as in the first embodiment are assigned, and their descriptions are omitted or simplified.

As shown in FIG. 6, when the CPU 12 starts medical information processing, it determines whether an instruction to specify the patient (target patient) whose medical information is to be processed has been input (S1). If the instruction specifying the target patient has not been input (S1: NO), the process of S1 is repeated and the state is maintained. When an instruction to specify the target patient is input (S1: YES), the CPU 12 specifies the patient to be processed for medical information according to the input instruction (S2). Next, the CPU 12, following the default display rule, determines the display order of multiple types of medical information related to the target patient (for example, in the present embodiment, S3 is the display order of all medical information for the target patient), and displays the multiple types of medical information in a matrix format on the display unit 17 according to the determined display order (S3).

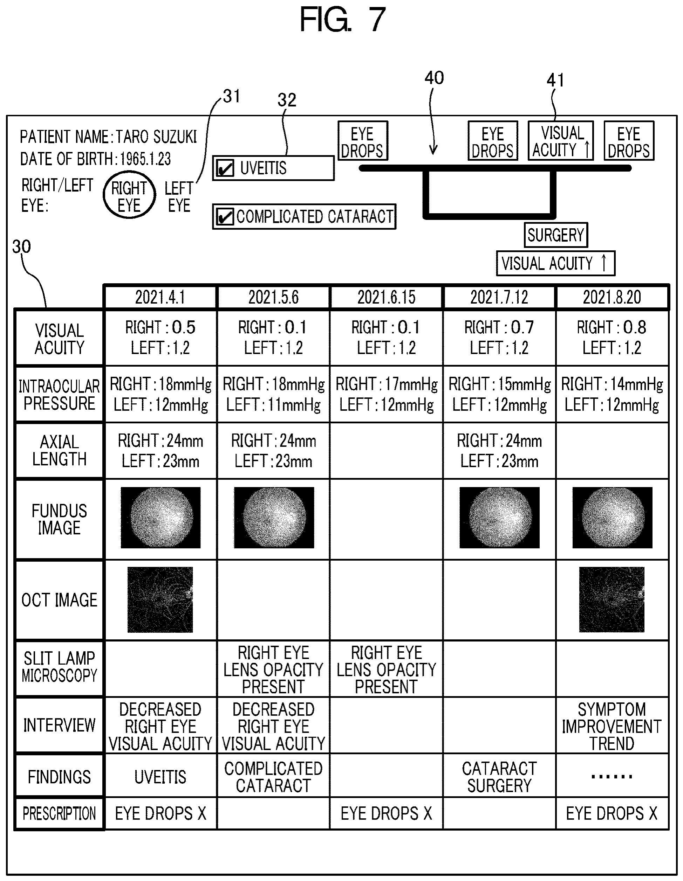

An example of the display of the matrix display section 30 as displayed by the processing of S3 in the second embodiment is shown in FIG. 7. As shown in FIG. 7, in the matrix display section 30, multiple types of medical information are arranged in one of the rows or columns (in FIG. 7, the rows) according to the display order (in FIG. 7, from top to bottom), and in the other column (in FIG. 7, the columns), each type of medical information is arranged and displayed in chronological order (in FIG. 7, from left to right). In addition, the user can operate the operation unit 16 and select either "right eye" or "left eye" in the eye selection section 31, thereby switching the medical information displayed in the matrix display section 30 between the right and left eyes.

In the upper central part of the screen shown in FIG. 7, a display rule selection section 32 is displayed. In the second embodiment, in the medical information processing device 10, multiple display rules for displaying multiple types of medical information in a matrix format are defined for each of a plurality of diseases. As described above, each display rule defines the rule for narrowing down the types of multiple types of medical information to be displayed in a matrix format, as well as the rule for determining the display order of multiple types of medical information. In other words, for each display rule, the narrowing rule for the types of medical information to be displayed in the matrix format, and the rule for determining the display order of the multiple types of medical information to be displayed in the matrix format, are set. The user operates the operation unit 16 to select (specify) one of the plurality of display rules, and the medical information is displayed on the screen 17 in accordance with the selected display rule. By displaying multiple types of medical information in a matrix format on the display unit in accordance with the display rule corresponding to the disease of the target patient, it becomes easier for medical professionals to appropriately perform medical care for the corresponding disease based on the displayed medical information. In the example shown in FIG. 7, display rules corresponding to each of the two diseases, "uveitis" and "complicated cataract," are shown. The number and content of the display rules corresponding to the diseases can be appropriately modified as necessary.

Returning to the explanation in FIG. 6. The CPU 12 displays disease progress information 40, which shows the course of a specific disease of the target patient in chronological order, based on the medical information regarding the specific disease of the target patient (S21). Accordingly, medical professionals can not only appropriately identify multiple types of medical information by means of the matrix display section 30, but can also easily comprehend the course of a specific disease by means of the disease progress information 40. Therefore, the efficiency and accuracy of patient treatment can be further improved.

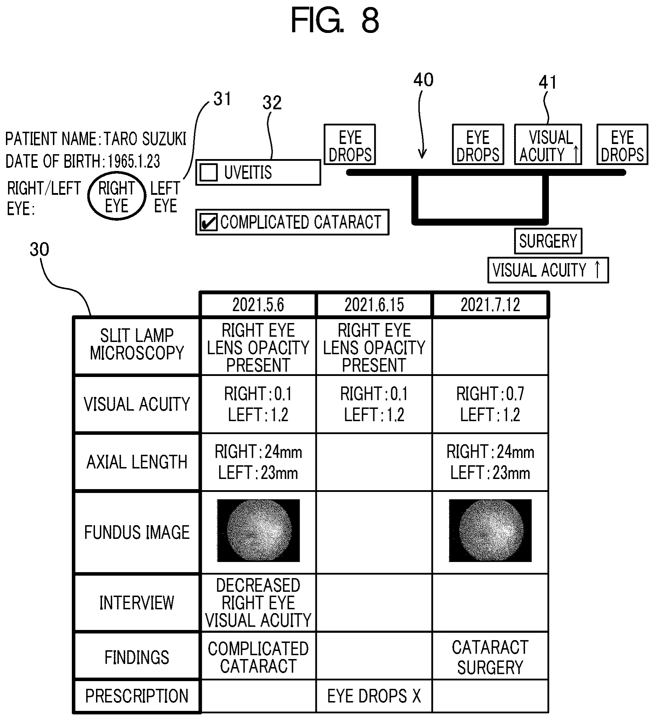

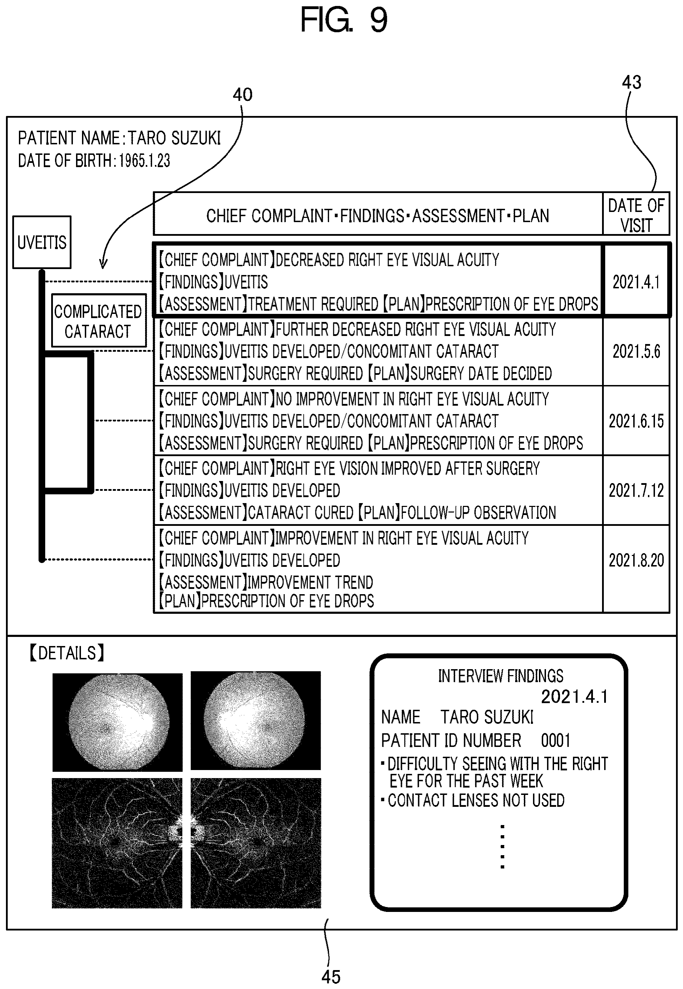

In the examples of FIG. 7 and FIG. 8, in the disease progress information 40 of the present embodiment, the course of the disease is indicated by a timeline along which a sequence of events progresses in a predetermined direction (in FIG. 7 and FIG. 8, to the right). In the example of FIG. 9, the course of the disease is indicated by a timeline along which a sequence of events progresses in the downward direction.

In addition, in the processing of S21 in the present embodiment, the CPU 12 displays, in the disease progress information 40, information indicating that an event related to a specific disease of the target patient has occurred (in the present embodiment, event icon 41), corresponding to the time in the chronological sequence at which the event occurred. The content of the event information to be displayed in the disease progress information 40 can be selected as appropriate. For example, at least one of the following may be displayed in the disease progress information 40 by means of icons, marks, comments, or the like: a visit event indicating that the target patient has visited the medical facility; a surgery event indicating that surgery has been performed on the target patient (the "surgery" icon in FIG. 7 and FIG. 8); a prescription event indicating that a prescription has been issued for the target patient (the "eye drops" icon in FIG. 7 and FIG. 8); a test event indicating that an examination (which may comprise imaging) has been performed on the target subject; a test result event indicating that the results of multiple examinations performed on the target patient satisfy a predetermined condition (for example, a condition in which the test result exceeds a threshold value, or a condition in which the amount of change in the test result exceeds a threshold value, or at least one of these conditions) (the "visual acuity ↑" icon in FIG. 7 and FIG. 8); and so on. For example, in the examples shown in FIG. 7 and FIG. 8, an event icon 41 for a test result event "visual acuity ↑," indicating that the increase in visual acuity from the previous measurement for a specific patient has exceeded a threshold value, is displayed. Accordingly, medical professionals can appropriately identify the timing at which the test result (in the examples shown in FIG. 7 and FIG. 8, the visual acuity test result) became noteworthy.

Furthermore, in the processing of S21 in the present embodiment, when there is a related disease that has occurred in connection with a specific disease, the CPU 12 displays disease progress information regarding the related disease (hereinafter referred to as "related disease progress information") branching from the chronological sequence of the disease progress information regarding the specific disease. Accordingly, medical professionals can appropriately identify the course of the related disease that has occurred in connection with the specific disease, in comparison with the course of the specific disease (hereinafter sometimes referred to as the "primary disease") that served as the basis for the occurrence of the related disease.

In the examples shown in FIGS. 7 to 9, uveitis is the primary disease, and the related disease, complicated cataract, has occurred. Accordingly, CPU 12 branches and displays the related disease progress information for the complicated cataract, which is a related disease, from the disease progress information for uveitis, which is the primary disease.

Details will be described later, but in the present embodiment, CPU 12 can, in response to instructions input by the user, branch related disease progress information from the primary disease progress information and display it. If the correspondence relationship between the primary disease and the related disease is predetermined, CPU 12 may automatically branch the related disease progress information from the primary disease progress information in accordance with the predetermined correspondence relationship. The period for branching the related disease history information from the disease history information of the underlying disease may be specified based on instructions input by the user, or may be automatically specified based on the period in which medical information relating to the related disease was acquired. The period for branching the related disease history information from the disease history information of the underlying disease may be corrected by the user if the actual occurrence of the related disease is later than the specified period.

In the processing of S21 in the present embodiment, when the treatment for a specific disease of the target patient has been completed, CPU 12 executes a process to stop the progress of the disease progress information series related to the specific disease, or a process to stop the progress of the series at the time when the treatment for the disease has been completed. As a result, when the medical care for a specific disease of the target patient is completed-such as by the cure of the disease or the transfer of the patient to another hospital (which may also be referred to as "transfer")-the time at which the medical care was completed is appropriately identified by the time at which the chronological progression of the disease progress information is stopped. Furthermore, the medical professionals can determine whether or not the progress of the disease progress information series has stopped, and can also determine whether or not the diagnosis and treatment for the disease are ongoing.

In the examples shown in FIGS. 7 to 9, when CPU 12 stops the chronological progression of the related disease progress information (in FIGS. 7 to 9, the disease progress information for complicated cataract), CPU 12 integrates the chronology of the related disease progress information into the chronology of the primary disease progress information (in FIGS. 7 to 9, the disease progress information for uveitis). In this case, medical professionals can appropriately identify that treatment of the related disease occurring in connection with the underlying disease has been completed by comparing it with the course of the underlying disease. Although not shown in FIGS. 7 to 9, the CPU 12 can also stop the chronological progression by stopping the extension of a line or the like that indicates the chronological progression of the disease progress information.

Returning to the explanation in FIG. 6. When the default matrix display of medical information and the display of disease course information are completed, the CPU 12 determines whether various setting instructions related to associated diseases (for example, at least one of instructions such as the setting of the correspondence relationship between the primary disease and associated diseases, and the setting of the time when the associated disease occurred) have been input by the user (S22). If not input (S22: No), the process proceeds to S25 as is. When setting instructions related to associated diseases are input (S22: YES), the CPU 12 is configured to perform display settings for the associated disease course information according to the instructions input by the user. For example, when an instruction to set the correspondence relationship between the primary disease and associated diseases is input, the CPU 12 executes a process to display the disease course information of the associated disease branching from the disease course information of the primary disease. In addition, when an instruction to set the time when the associated disease occurred is input, the CPU 12 sets the timing for branching the disease course information of the associated disease from the disease course information of the primary disease to the time specified by the instruction.

Next, the CPU 12 determines whether a setting instruction to terminate the treatment for a specific disease has been input by the user (S25). If not input (S25: No), the process proceeds to S5 as is. When a setting instruction to terminate the treatment for a specific disease is input (S25: YES), the CPU 12 executes a time series progression stop process (S26) that stops the progression of the time series of the disease course information for the specified disease. As previously described, in S26, if the specified disease is an associated disease, the CPU 12 executes a process to integrate the time series of the associated disease course information into the time series of the primary disease course information. Additionally, the CPU 12 may automatically acquire the timing at which the treatment for the disease was completed based on medical information related to the disease, and stop the progression of the time series of the disease course information accordingly.

Next, the CPU 12 determines whether at least one of the multiple display rules has been specified (S5). In the second embodiment, the display rule is specified when the user checks the checkbox within the desired display rule selection section 32 among the multiple display rule selection sections 32, each corresponding to a different disease. When at least one of the multiple display rules is specified (selected) (S5: YES), the CPU 12 sets the specified display rule as the display rule for performing display information adjustment processing (S6). The CPU 12 executes display information adjustment processing according to the set display rule (that is, at least one of narrowing down the types of multiple kinds of medical information for the target patient and determining the display order) (S12). The CPU 12, in accordance with the result of the display information adjustment processing, displays multiple types of medical information in a matrix format (S13).

FIG. 8 illustrates an example of a screen in which the matrix display of medical information has been changed according to the display rule corresponding to the associated disease, secondary cataract, starting from the default display state shown in FIG. 7 (the state in which the medical information for both uveitis and secondary cataract is displayed in a matrix format). In the example shown in FIG. 8, among the multiple types of medical information, several items that are highly important for the treatment of secondary cataract are selected. These items are then arranged in order of importance for the treatment of secondary cataract, from top to bottom, and displayed in the matrix display section 30.

Next, the CPU 12 executes a process to switch the display between the matrix display section 30 for multiple types of medical information (see FIG. 7 and FIG. 8) and the time-based display section 43 (see FIG. 9), according to instructions input by the user (S28). In the following, the time-based display section 43 in the second embodiment will be described with reference to FIG. 9. In the second embodiment, the CPU 12 can display one or more pieces of medical information for each time point indicated by the time series of disease course information 40, in the time-based display section 43. In the time-based display section 43 shown in FIG. 9, for each time point indicated by the time series of the disease course information 40, the chief complaint, findings, assessment, and plan comprised in the medical information are displayed. Accordingly, medical professionals can sequentially identify the medical information of the target patient at each corresponding time point, in accordance with the time series of the disease course information 40.

In this embodiment, when the user inputs an instruction specifying at least one of the multiple time points at which medical information has been obtained, the CPU 12 can collectively display the medical information acquired at the specified time in the detailed display section 45. In the example shown in FIG. 9, since the consultation date "2021.4.1" has been specified by the user, the CPU 12 displays the medical information obtained on "2021.4.1" in the detailed display section 45. In this case, medical professionals can appropriately confirm the details of the medical information for the specified time while identifying the multiple pieces of medical information in chronological order. Thus, the efficiency and accuracy of patient treatment can be further improved.

### (Third embodiment)

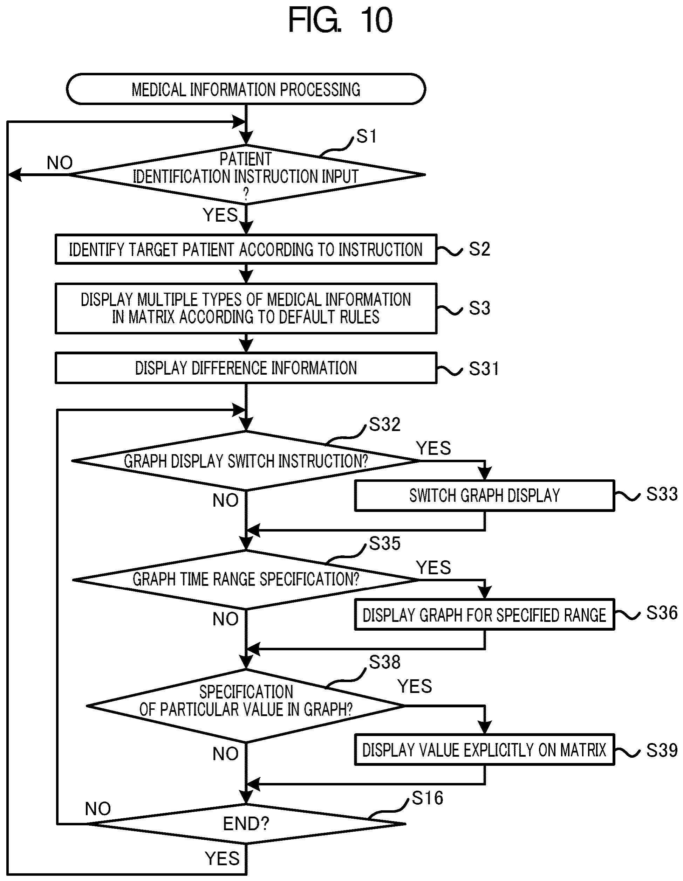

Referring to FIG. 10 to FIG. 13, the medical information processing performed by the medical information processing device 10 of the third embodiment will be described. In the medical information processing of the third embodiment (see FIG. 10), multiple types of medical information are displayed in a matrix format, and for at least some types of medical information whose values may increase or decrease, value transition information indicating the changes in values over time is displayed on the display unit 17 together with the matrix display. The medical information processing is executed by the CPU 12 of the medical information processing device 10 in accordance with the medical information processing program stored in the storage device 13. In the third embodiment, at least some of the steps of the medical information processing may employ the same processing as the steps of the medical information processing in the aforementioned first and second embodiments. Accordingly, for steps that can employ the same processing as the steps in the first and second embodiments, the same step numbers as in the first and second embodiments are assigned, and their descriptions are omitted or simplified.

As shown in FIG. 10, when the CPU 12 starts medical information processing, it determines whether an instruction to specify the patient (target patient) whose medical information is to be processed has been input (S1). If the instruction specifying the target patient has not been input (S1: NO), the process of S1 is repeated and the state is maintained. When an instruction to specify the target patient is input (S1: YES), the CPU 12 specifies the patient to be processed for medical information according to the input instruction (S2). Next, the CPU 12, following the default display rule, determines the display order of multiple types of medical information related to the target patient (for example, in the present embodiment, S3 is the display order of all medical information for the target patient), and displays the multiple types of medical information in a matrix format on the display unit 17 according to the determined display order (S3).

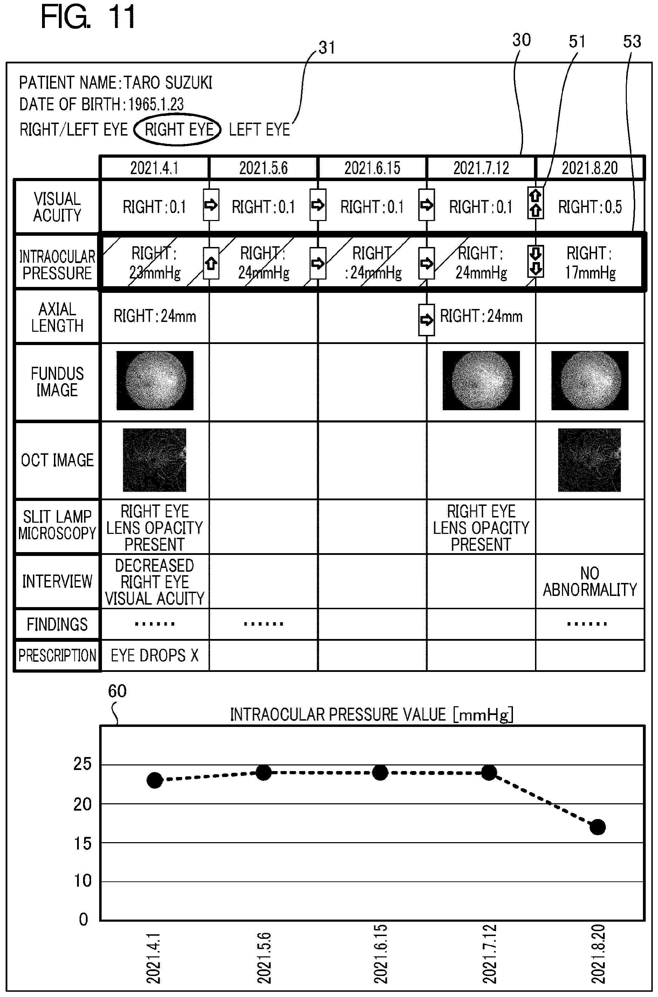

Examples of the matrix display section 30 as displayed by the processing in S3 of the third embodiment are shown in FIG. 11 to FIG. 13. In the matrix display section 30 of the third embodiment, as in the first and second embodiments, multiple types of medical information are arranged in order of display in either the vertical or horizontal rows, and the other columns display each type of medical information arranged in chronological order. In addition, the user can operate the operation unit 16 and select either "right eye" or "left eye" in the eye selection section 31, thereby switching the medical information displayed in the matrix display section 30 between the right and left eyes. Furthermore, in the third embodiment as well, as in the first and second embodiments, after the display information adjustment processing according to the set display rules has been executed, multiple types of medical information may be displayed in a matrix format.

Next, the CPU 12 displays difference information for at least some types of medical information whose values may increase or decrease, by adding this information to the matrix display section 30 (S31). Difference information refers to information that indicates at least one of the following for at least some values of medical information: the difference from the value of the same type of medical information obtained previously, and the difference from a reference value. Difference information is an example of value transition information that shows the changes in the values of medical information over time. According to the third embodiment, medical professionals can appropriately identify the transitions in the values of medical information, while identifying multiple types of medical information through the matrix display section 30, by means of the difference information added to the matrix display section 30.

In the examples shown in FIG. 11 to FIG. 13, for each of the medical information items displayed in the matrix display section 30 whose values may increase or decrease-specifically, "visual acuity," "intraocular pressure," and "axial length"-a difference icon 51 is additionally displayed as difference information, indicating the difference from the value of the same type of medical information obtained previously. The specific display style of the difference icon 51 can be selected as appropriate. The specific display style of the difference icon 51 can be selected as appropriate. In the examples shown in FIG. 11 to FIG. 13, when the value is higher than the previous value, the CPU 12 displays an upward arrow; when the value is lower, a downward arrow is displayed; and when the value is the same, a horizontal (rightward in FIG. 11 to FIG. 13) arrow is displayed. Furthermore, the greater the difference, the more arrows are displayed. Specifically, if the difference is equal to or greater than the threshold, two arrows are displayed; if the difference is less than the threshold, one arrow is displayed. It is also possible to vary the size of the arrows according to the magnitude of the difference.

In the examples shown in FIG. 11 to FIG. 13, among the values of medical information displayed in the matrix display section 30, for axial length values whose difference from the reference value is positive (in FIG. 11 to FIG. 13, intraocular pressure values greater than the reference value of 18 mmHg), difference information indicating that the value is greater than the reference value is additionally displayed by a change in the color within the frame (represented by hatching in FIG. 11 to FIG. 13). In this case, medical professionals can easily determine whether each value is greater than the reference value by checking the color within the frame in the matrix display section 30.

Returning to the explanation in FIG. 10. The CPU 12 determines whether the display switching instruction of the graph 60 has been inputted by a user (S32). In this embodiment, graph 60 is a graphical representation of the values of at least some types of medical information. Graph 60 is an example of value transition information that shows changes in the values of medical information over time. If a display switching instruction has not been input (S32: NO), the process proceeds to S35 as is. When the user operates the operation unit 16 and a display switching instruction for graph 60 is input (S32: YES), the CPU 12 executes a process to switch the display of graph 60 according to the input instruction (S33).

For example, by operating the operation unit 16, the user can specify, on the matrix display section 30, the type of medical information among multiple types for which they wish to view graph 60. In the examples shown in FIG. 11 to FIG. 13, when "intraocular pressure" is specified on the matrix display section 30, the frame for intraocular pressure (by default, the entire frame containing all values) is indicated as the designated frame 53 with a bold outline. When a type of medical information is specified on the matrix display section 30 (S32: YES), the CPU 12 displays, on the display unit 17, graph 60 showing the transitions in the values of the specified medical information. In the examples of FIG. 11 to FIG. 13, the graph 60 for "intraocular pressure" specified by the user is displayed on the display unit 17 together with the matrix display section 30. According to this embodiment, medical professionals can identify multiple types of medical information on the matrix display section 30 and, by directly specifying the medical information for which they wish to check value transitions on the matrix display section 30, can view the graph 60 for the specified medical information. Furthermore, when the user changes the specification of the medical information for which the graph is displayed (that is, changes the designated frame 53 to the frame of another type of medical information), the CPU 12 switches the display of graph 60 to the graph of the newly specified medical information.

The user can input an instruction to switch the display of graph 60 on or off by operating the operation unit 16. When an instruction to hide graph 60 is input (S32: YES), the CPU 12 terminates the display of graph 60 on the display unit 17.

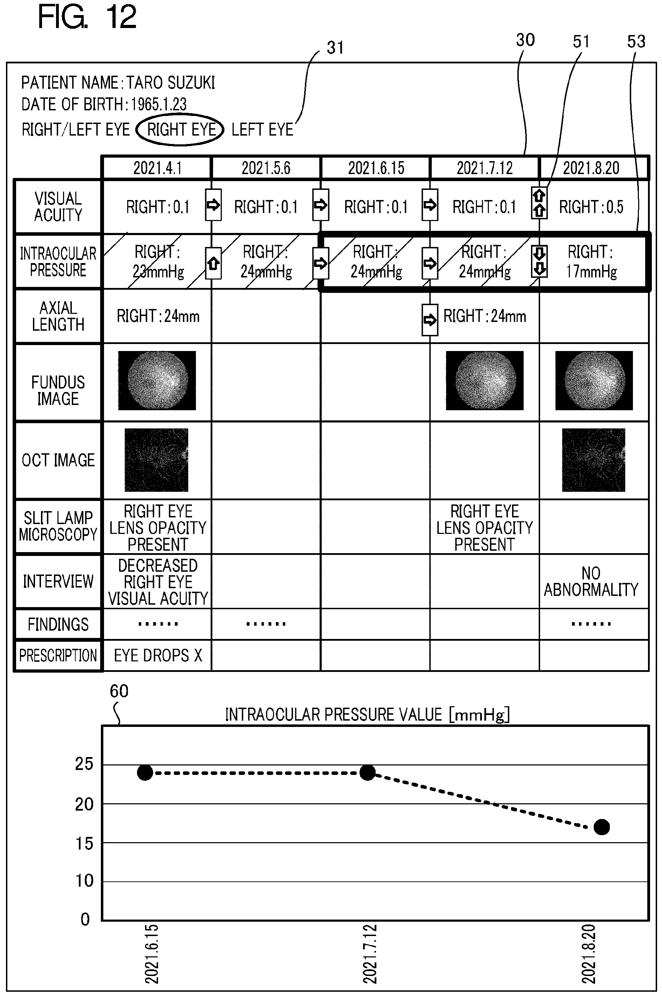

Next, the CPU 12 determines whether a time range for displaying graph 60 has been specified on the matrix display section 30 (S35). In this embodiment, the user can specify (or change) the time range to be comprised in the designated frame 53 by operating the operation unit 16, thereby specifying the time range for displaying graph 60 on the matrix display section 30. If the time range has not been specified (S35: NO), the process proceeds to S38 as is. When the time range is specified (S35: YES), the CPU 12 displays, on the display unit 17, graph 60 showing the transitions in the values of the medical information within the specified time range on the matrix display section 30 (S36). Accordingly, medical professionals can confirm graph 60 for the specified time range of medical information by specifying the time range on the matrix display section 30, where multiple types of medical information are shown. FIG. 12 illustrates an example of a screen when the time range for displaying graph 60 has been changed (specified) on the matrix display section 30 from the display state shown in FIG. 11. In the example shown in FIG. 12, the time range for displaying graph 60 has been changed from the entire range to a partial range. As a result, graph 60 for only the specified partial time range is displayed on the display unit 17.

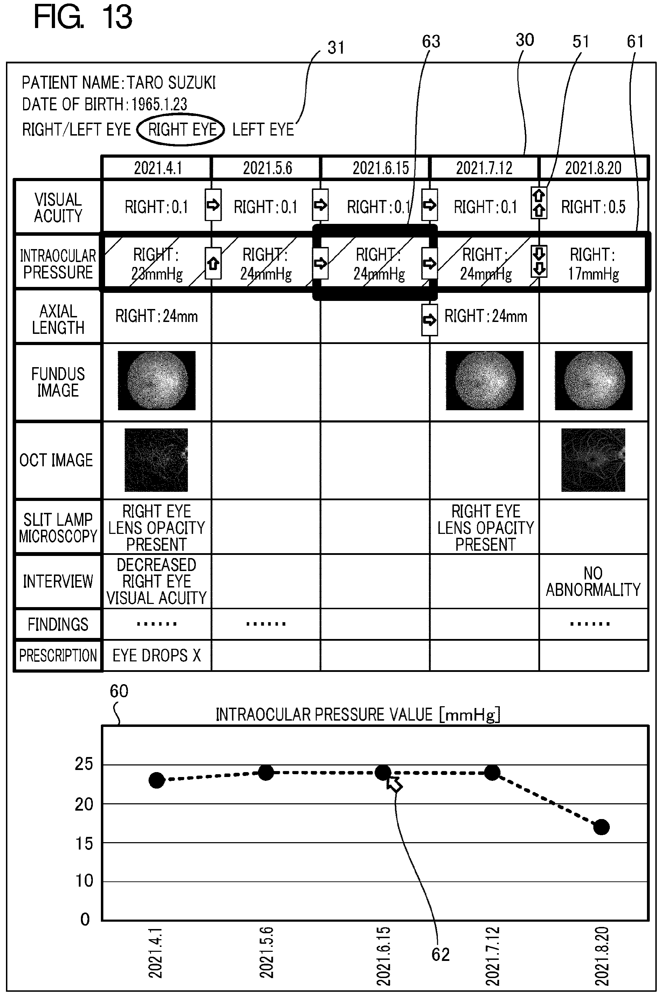

Next, the CPU 12 determines whether a specific value on the displayed graph 60 has been specified by the user (S38). In this embodiment, the user can specify a particular value on graph 60 by operating the operation unit 16 and moving the pointer shown in FIG. 13 to the desired value and clicking it. If a specific value has not been specified (S38: NO), the process proceeds to S16 as is. When a specific value is specified on graph 60 (S38: YES), the CPU 12 executes a process to highlight the value specified on graph 60 among the multiple values displayed in the matrix display section 30 (S39). Accordingly, medical professionals can easily confirm the specified value on the matrix display section 30 simply by specifying the value on graph 60. For example, it is also possible to compare the value specified on graph 60 with other types of medical information obtained at the same time on the matrix display section 30 for clinical purposes. FIG. 13 shows an example of a screen when a specific value on graph 60 (the intraocular pressure value of the right eye obtained on June 15, 2012) is specified, starting from the display state shown in FIG. 11. In the example shown in FIG. 13, the value specified on graph 60 is highlighted on the matrix display section 30 by a bold frame 63. As a result, the value specified on graph 60 can be easily identified on the matrix display section 30.

### (Fourth embodiment)

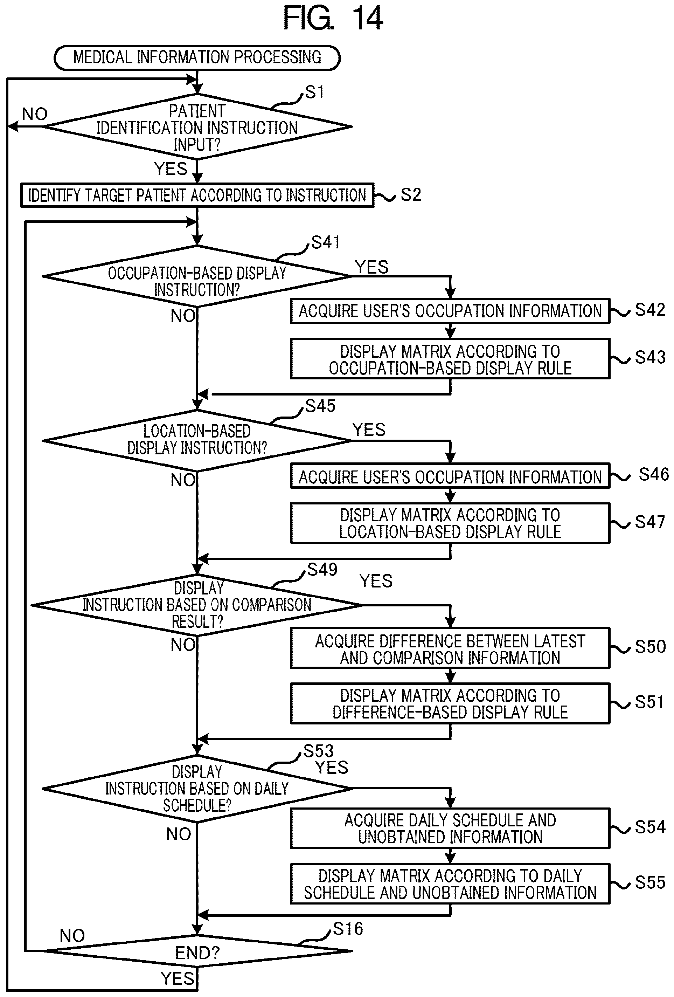

Referring to FIG. 14 to FIG. 17, the medical information processing performed by the medical information processing device 10 of the fourth embodiment will be described. In the medical information processing of the fourth embodiment (see FIG. 14), the display rules for multiple types of medical information are set based on status information regarding at least one of the usage status of the medical information processing device 10 and the status of the target patient. According to the set display rules, multiple types of medical information are displayed in a matrix format. The medical information processing is executed by the CPU 12 of the medical information processing device 10 in accordance with the medical information processing program stored in the storage device 13. It is also possible for at least some of the steps in the medical information processing of the fourth embodiment to employ the same processing as the steps in the medical information processing of the aforementioned first to third embodiments. Accordingly, for steps that can employ the same processing as the steps in the first to third embodiments, the same step numbers as in the first to third embodiments are assigned, and their descriptions are omitted or simplified.

In the medical information processing of the fourth embodiment, the display rules for multiple types of medical information are set based on at least one of various types of status information. In the example shown in FIG. 14, at least one of the following is referenced as status information: occupation information, usage location information, difference information, and schedule information for the day (including unacquired information). The user can operate the operation unit 16 to input into the medical information processing device 10 which of the multiple types of status information should be used as the basis for setting the display rules.

As shown in FIG. 14, when the CPU 12 starts medical information processing, it determines whether an instruction to specify the patient (target patient) whose medical information is to be processed has been input (S1). If the instruction specifying the target patient has not been input (S1: NO), the process of S1 is repeated and the state is maintained. When an instruction to specify the target patient is input (S1: YES), the CPU 12 specifies the patient to be processed for medical information according to the input instruction (S2).

Next, the CPU 12 determines whether a display instruction for the matrix display section 30 based on occupation information (that is, an occupation-specific display instruction) has been input by the user (S41). Occupation information refers to information that can identify the occupation of the user operating the medical information processing device 10. When a display instruction by occupation is input (S41: YES), the CPU 12 acquires occupation information that makes it possible to identify the occupation of the user using the medical information processing device 10 (for example, at least one of physician, nurse, pharmacist, radiological technologist, physical therapist, occupational therapist, clinical psychologist, certified orthoptist (CO: Certified Orthoptist), nutritionist, or the like) (S42). The specific form of the occupation information acquired in S42 can be selected as appropriate. For example, when the user logs in to the medical information processing device 10, various login information (for example, at least one of user ID, user name, etc.) may comprise information indicating the user's occupation. Additionally, the login information may be associated with the occupation of the user. In this case, the CPU 12 may acquire the login information of the user logged into the medical information processing device 10 as occupation information that can identify the occupation. Furthermore, the user may input information indicating their own occupation into the medical information processing device 10 via the operation unit 16 or the like. In this case, the information input via the operation unit 16 or the like may be used as occupation information.

The CPU 12 sets the display rules for multiple types of medical information to be shown in the matrix display section 30 according to the occupation of the user, as identified by the occupation information obtained in S42. Following the display rules set for each occupation, the CPU 12 is configured to perform display information adjustment processing in the same manner as in the first to third embodiments, and then displays multiple types of medical information in a matrix format on the display unit 17 (S43). In many cases, the importance of each type of medical information differs depending on the occupation of the user of the medical information processing device 10. Therefore, by following display rules corresponding to the occupation, multiple types of medical information can be displayed in a matrix format, making it easier to appropriately present necessary medical information to each medical professional.

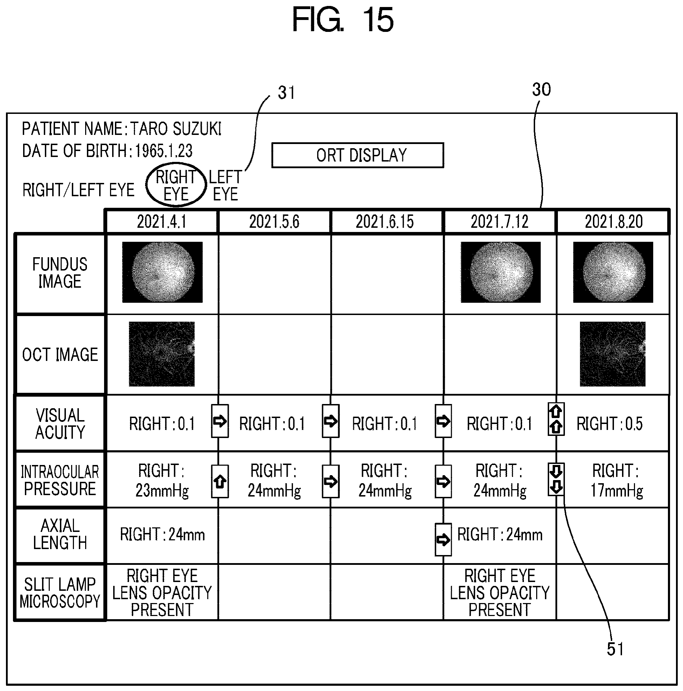

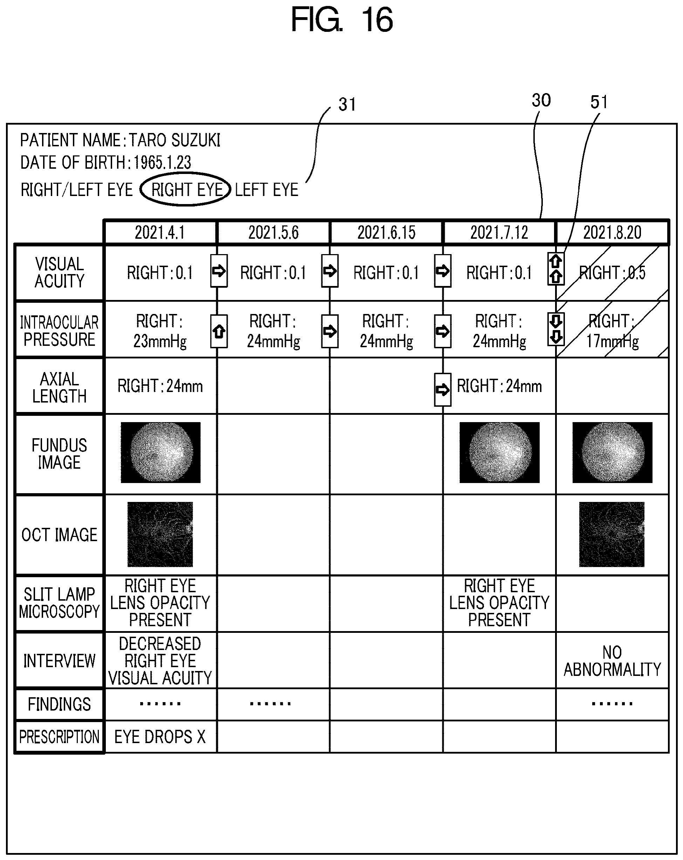

An example of the display of the matrix display section 30 as shown by the processing of S43 is illustrated in FIG. 15. The matrix display section 30 in FIG. 15 illustrates the case where the user's occupation is a certified orthoptist (CO). Unlike physicians and others, certified orthoptists have fewer opportunities to refer to medical information such as interviews and findings. On the other hand, certified orthoptists need to confirm whether information such as ophthalmic images, OCT images, and other test results necessary for medical treatment by physicians has been appropriately obtained. Accordingly, in the example shown in FIG. 15, the display rules are set so that, among the multiple types of medical information for the target patient, information such as test results is selected and displayed in the matrix display section 30. Furthermore, the display rules are set so that the display order of multiple types of medical information is determined according to the importance of confirming the acquired test results. In addition, in the fourth embodiment, as in the third embodiment, a difference icon 51 indicating the difference from the value of the same type of medical information obtained previously is additionally displayed as difference information (see FIG. 15 and FIG. 16).

Next, the CPU 12 determines whether a display instruction for the matrix display section 30 based on usage location information (that is, a location-specific display instruction) has been input by the user (S45). Usage location information refers to information that can identify the location where the medical information processing device 10 is being used. If a location-specific display instruction has been input (S45: YES), the CPU 12 acquires usage location information that can identify the location where the medical information processing device 10 is being used (for example, at least one of various examination rooms, consultation rooms, operating rooms, reception areas within a medical facility, and locations outside the medical facility such as home visit sites) (S46). The specific form of the usage location information acquired in S46 can be selected as appropriate. For example, when the medical information processing device 10 is used in a specific location, usage location information indicating the usage location may be set for each medical information processing device 10. In this case, the CPU 12 may acquire the usage location information set in the medical information processing device 10 from the storage device 13 or the like. Additionally, when the medical information processing device 10 can be used in multiple locations, the user may input information indicating the usage location into the medical information processing device 10 via the operation unit 16 or the like. In this case, the information input via the operation unit 16 or the like may be used as usage location information. Furthermore, if the medical information processing device 10 is capable of acquiring location information, the CPU 12 may acquire the obtained location information as usage location information that can identify the usage location.

The CPU 12 sets the display rules for multiple types of medical information to be shown in the matrix display section 30, according to the usage location of the medical information processing device 10 as identified by the usage location information obtained in S46. The CPU 12 is configured to perform display information adjustment processing in the same manner as in the first to third embodiments, according to the display rules set for each usage location, and then displays multiple types of medical information in a matrix format on the display unit 17 (S47). The importance of each type of medical information often differs depending on the location where the medical information processing device 10 is used. Accordingly, by displaying multiple types of medical information in a matrix format according to the display rules corresponding to the usage location of the medical information processing device 10, it becomes easier to appropriately present the necessary medical information to medical professionals engaged in medical care at each location.

Next, the CPU 12 determines whether a display instruction for the matrix display section 30 based on difference information (that is, a display instruction according to the comparison result) has been input by the user (S49). The difference information is obtained for at least some of the multiple types of medical information of the target patient. The difference information indicates the difference between the latest medical information (latest information) obtained for the target patient and the same type of medical information used as a comparison reference (comparison information). For example, when the medical information is numerical data such as test results, or numerical data obtained from captured image information, the difference information may be represented by numerical values. If a display instruction according to comparison results has been input (S49: YES), the CPU 12 obtains difference information indicating the difference between the latest information and the comparison information for at least some of the multiple types of medical information of the target patient (S50). The CPU 12 sets the display rules for multiple types of medical information to be shown in the matrix display section 30 according to the difference information obtained in S50. Following the display rules set according to the difference information, the CPU 12 is configured to perform display information adjustment processing in the same manner as in the first to third embodiments, and then displays multiple types of medical information in a matrix format on the display unit 17 (S51). By setting the display rules for multiple types of medical information based on the difference information indicating the difference between the latest information and the comparison information, it becomes easier for the user to appropriately identify the condition of the target patient.

An example of the matrix display section 30 as displayed by the processing in S51 is shown in FIG. 16. In the example shown in FIG. 15, the CPU 12 sets the display rules so that, among at least some of the multiple types of medical information for the target patient, the greater the difference indicated by the difference information, the higher the row in which the information is displayed among the multiple horizontal rows. As a result, the medical information is arranged and displayed in order from the top according to the magnitude of the difference relative to the comparison information. Therefore, users can easily identify the types of medical information that have the largest differences from the reference information. In the example shown in FIG. 16, focusing on the magnitude of the difference between the latest information and the comparison information, visual acuity has the largest difference, and intraocular pressure has the second largest difference. As a result, among the multiple types of medical information, visual acuity is displayed in the topmost row, and intraocular pressure is displayed in the second row from the top.

In the example shown in FIG. 16, the CPU 12 sets the display style on the display unit 17 for medical information whose difference indicated by the difference information is equal to or greater than the threshold (for example, the color within the frame, the color of the frame, the color of the text, the presence or absence of a mark, etc.) to be different from the display style for medical information whose difference is less than the threshold. In this case, the user can more easily identify medical information for which the difference from the comparison information is equal to or greater than the threshold, based on the display style of the medical information. Specifically, in the example shown in FIG. 16, the color within the frame for medical information (visual acuity and intraocular pressure) whose difference indicated by the difference information is equal to or greater than the threshold is set to a color different from that of the other medical information.

When setting display rules for multiple types of medical information according to the magnitude of the difference indicated by the difference information, the specific form of the difference information can be set as appropriate. As one example, in this embodiment, the CPU 12 obtains, as difference information, deviations of the latest information from the comparison information for at least some of the multiple types of medical information. The CPU 12 sets the display rules so that the greater the obtained deviation for a particular type of medical information, the higher the row in which that information is displayed among the multiple horizontal rows. In this case, multiple types of medical information with mutually different deviations are displayed in the display unit in an appropriate order according to the deviation.

It is also possible to modify the method for setting display rules based on difference information. For example, the CPU 12 may display one or more types of medical information for the target patient, for which the difference indicated by the difference information is equal to or greater than the threshold, in higher rows among the multiple horizontal rows, compared to one or more types of medical information for which the difference is less than the threshold. Even in this case, the user can easily identify medical information for which the difference from the comparison information is equal to or greater than the threshold by checking the upper rows of the matrix display section 30. When using a threshold, the difference information may be the deviation described above, or it may be the difference in value between the latest information and the comparison subject information. The threshold may be set for each type of medical information.

In S50 of this embodiment, the CPU 12 acquires, as status information, difference information indicating the difference between the latest medical information (latest information) obtained for at least one of multiple types of medical information of the target patient, and comparison information, which is the same type of medical information obtained for the same target patient immediately prior to the latest information. When the comparison information is medical information obtained at the previous consultation for the same target patient (hereinafter referred to as "previous information"), the greater the difference between the latest information and the previous information, the higher the likelihood that the patient's condition has changed (worsened or improved). Accordingly, by setting display rules for multiple types of medical information based on the difference information indicating the difference between the latest information and the previous information, it becomes easier for the user to appropriately identify the condition of the target patient.

However, it is also possible to change the specific form of the comparison information. For example, medical information of a healthy person without disease (for example, at least one of normal values, average values, permissible values, and permissible ranges) may be used as the comparison information. Alternatively, without limiting the comparison subject information to the medical information obtained immediately prior to the latest information, medical information of the same type obtained for the same subject on a specific day may be used as the comparison subject information. In this case as well, the condition of the patient can be more appropriately identified by the user. In addition, the control unit may obtain, as difference information, the rate of change (for example, the slope of values, etc.) of the same type of medical information for the same target patient, obtained over multiple occasions (for example, three or more times), including the latest medical information. The control unit may also set display rules according to the acquired difference information. For example, the control unit may display, in the upper row of multiple horizontal rows or in the leftmost column of multiple vertical columns, the medical information for which the rate of change between the latest information and the previous information is greater than or equal to the rate of change before the previous information (for example, the average rate of change before the previous information, etc.). Alternatively, the control unit may set display rules so that the greater the difference from the rate of change between the latest information and the previous information (for example, the average rate of change before the previous information, etc.), the higher the medical information is displayed in the upper row of multiple horizontal rows or the further left in the leftmost column of multiple vertical columns. In this case, the medical information for which the rate of change becomes greater can be more easily identified by the user.

Next, the CPU 12 determines whether a display instruction for the matrix display section 30 based on schedule information for the day (that is, a display instruction according to the schedule for the day) has been input by the user (S53). Schedule information for the day refers to information indicating which types of medical information are scheduled to be obtained for the target patient on that day, among multiple types of medical information. In addition, in the fourth embodiment, the schedule information for the day also comprises unacquired information, which indicates which types of medical information scheduled to be obtained for the target patient on that day have not yet been obtained. If a display instruction according to the schedule for the day has been input (S53: YES), the CPU 12 acquires the schedule information for the day and the unacquired information for the target patient (S54). The schedule information for the day may be input by the user operating the operation unit 16, or it may be acquired based on information from the electronic medical record or reservation information, for example. The CPU 12 sets the display rules for multiple types of medical information to be shown in the matrix display section 30 according to the schedule information for the day acquired in S54. Following the display rules set according to the schedule information for the day, the CPU 12 is configured to perform display information adjustment processing in the same manner as in the first to third embodiments, and then displays multiple types of medical information in a matrix format on the display unit 17 (S55).

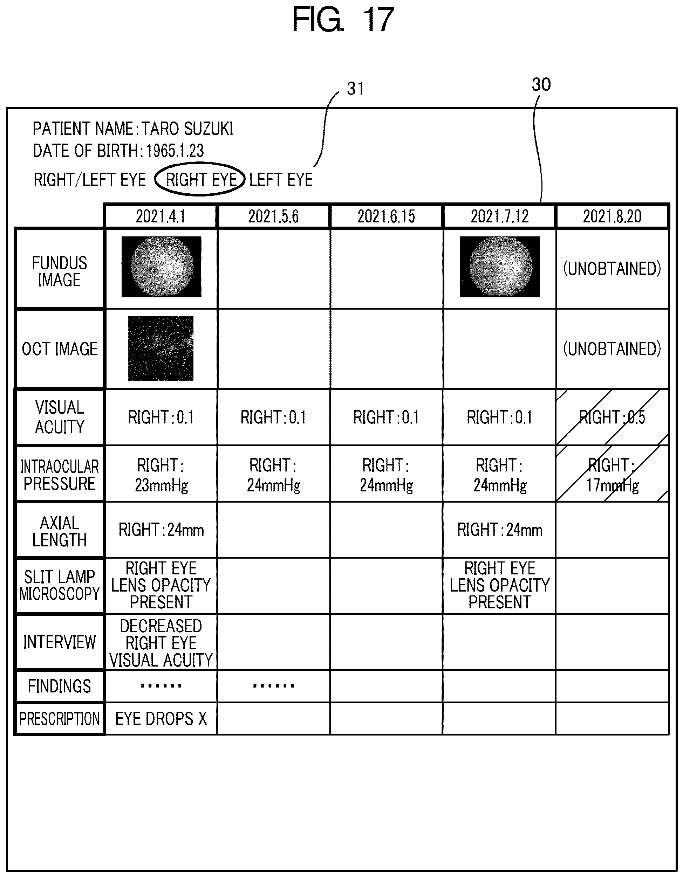

An example of the matrix display section 30 as displayed by the processing in S55 is shown in FIG. 17. In the example shown in FIG. 17, the CPU 12 displays the items of medical information that have not yet been obtained on that day, as indicated by the unacquired information (in FIG. 17, fundus images and OCT images), in the upper rows among the multiple horizontal rows, compared to the other items of medical information. Accordingly, the user can easily identify the medical information that has not yet been obtained among the medical information scheduled to be obtained for the target patient on that day, by checking the upper rows in the matrix display section 30.

In the example shown in FIG. 17, the CPU 12 sets the display style on the display unit 17 for items of medical information that have not yet been obtained on that day (for example, the presence or absence of explanatory text, the color of the frame, the color within the frame, the color of the text, the presence or absence of a mark, etc.) to be different from the display style for other medical information. Accordingly, the user can more easily identify, by means of the display style of the medical information, which items of medical information scheduled to be obtained on that day have not yet been obtained. As one example, in the case shown in FIG. 17, only the items of medical information that have not yet been obtained on that day are labeled with the text "unobtained".

In the example shown in FIG. 17, the CPU 12 displays the items of medical information scheduled to be obtained for the target patient on that day-including both information already obtained on the day ("visual acuity," "intraocular pressure") and information not yet obtained on the day ("fundus images," "OCT images")-in the upper rows among the multiple horizontal rows, compared to other items of medical information. Accordingly, by checking the upper rows, the user can easily identify both the medical information scheduled to be obtained for the target patient on that day and the medical information that has already been obtained on that day. Furthermore, the items of medical information that have not yet been obtained on the day are displayed above the items of medical information that have already been obtained on the day. As a result, it becomes easier to appropriately identify the progress of the day's clinical activities.

In the example shown in FIG. 17, the CPU 12 sets the display style on the display unit 17 for items of medical information that have already been obtained on that day (for example, the presence or absence of explanatory text, the color of the frame, the color within the frame, the color of the text, the presence or absence of a mark, etc.) to be different from the display style for other medical information. Accordingly, the user can more easily identify the medical information that has been obtained on that day by means of the display style of the medical information. As one example, in the case shown in FIG. 17, the color within the frame for items of medical information that have been obtained on that day is set to a color different from that of other items.

### (Fifth embodiment)

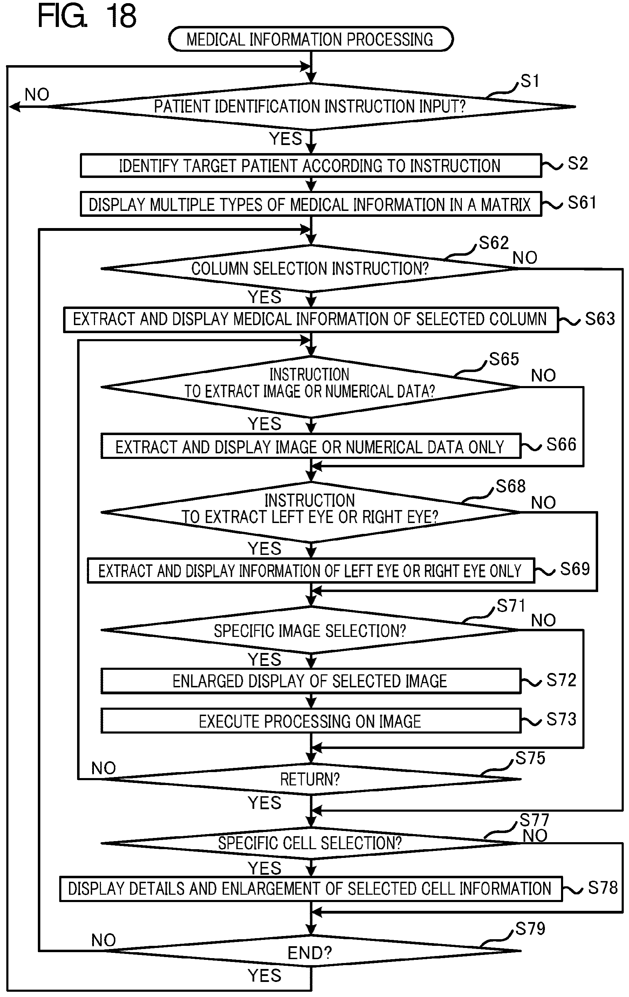

Referring to FIG. 18 to FIG. 21, the medical information processing performed by the medical information processing device 10 of the fifth embodiment will be described. In the medical information processing of the fifth embodiment (see FIG. 18), multiple types of medical information are displayed in a matrix format. In this state, at least one column (vertical or horizontal) in the matrix display section 30 is selected by the user. When a column is selected, the medical information that was displayed in the selected column is extracted from the medical information of the target patient, and the extracted medical information is displayed on the display unit 17. The medical information processing is executed by the CPU 12 of the medical information processing device 10 in accordance with the medical information processing program stored in the storage device 13. In the fifth embodiment, at least some of the steps of the medical information processing may employ the same processing as the steps of the medical information processing in the aforementioned first to fourth embodiments. Accordingly, for steps that can employ the same processing as the steps in the first to fourth embodiments, the same step numbers as in the first to fourth embodiments are assigned, and their descriptions are omitted or simplified.

As shown in FIG. 18, when the CPU 12 starts medical information processing, it determines whether an instruction to specify the patient (target patient) whose medical information is to be processed has been input (S1). If the instruction specifying the target patient has not been input (S1: NO), the process of S1 is repeated and the state is maintained. When an instruction to specify the target patient is input (S1: YES), the CPU 12 specifies the patient to be processed for medical information according to the input instruction (S2).

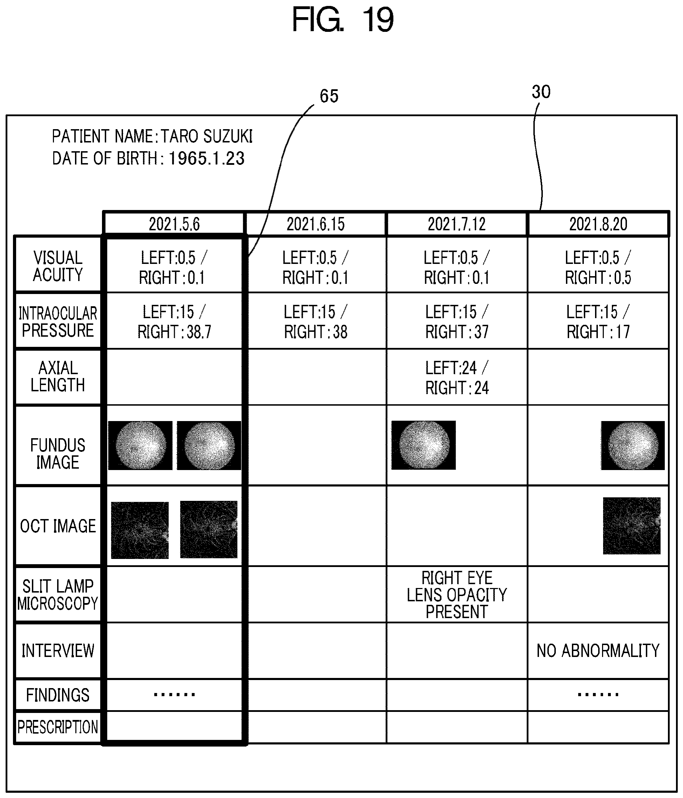

Next, the CPU 12 displays multiple types of medical information in a matrix format on the display unit 17 (S61). As shown in FIG. 19, in the matrix display section 30, multiple types of medical information are arranged in one set of columns (in FIG. 19, the horizontal rows) according to the display order (from top to bottom in FIG. 19). In the other set of columns (in FIG. 19, the vertical columns), each type of medical information is arranged and displayed in chronological order (from left to right in FIG. 19). Therefore, by referring to the matrix display 30, medical professionals can appropriately identify multiple types of medical information related to the target patient in chronological order.

In S61, the CPU 12 is configured to perform excerpt processing for part of the information or reduction processing for images on at least some of the multiple types of medical information for the target patient, and displays the resulting simplified information in a matrix format on the display unit 17. As a result, the user can appropriately identify multiple types of medical information based on the simplified information, which is displayed in a matrix format after being excerpted or reduced. For example, in the case shown in FIG. 19, for "intraocular pressure," each value measured multiple times for one eye is not displayed; instead, only the average value of the multiple measurements is excerpted and displayed in the matrix display section 30. In the example shown in FIG. 19, for "fundus images" and "OCT images," only representative images among the multiple captured images are reduced in size and displayed as thumbnails in the matrix display section 30.

In the example shown in FIG. 19, among the tissues present on the left and right sides of the target patient, the medical information obtained for the left tissue (in this embodiment, the left eye) is displayed on the left side within the frame of the matrix display section 30. Similarly, the medical information obtained for the right tissue (in this embodiment, the right eye) is displayed on the right side within the frame of the matrix display section 30. When medical information for both the left and right tissues of the same target patient has been obtained, the information for the left tissue is displayed on the left side and the information for the right tissue is displayed on the right side within the same frame of the matrix display section 30. Therefore, the user can easily and appropriately identify whether each piece of medical information displayed in the matrix display section 30 pertains to the left or right tissue, based on the display position of each item within the frame.

In S61, the specific details of the display information adjustment processing-such as the filtering process and the determination of display order-when displaying multiple types of medical information in a matrix format can be selected as appropriate. For example, in S61, multiple types of medical information may be displayed in a matrix according to the default display rules, as described previously in S3 (see FIG. 3, FIG. 6, and FIG. 10). In S61, the adjustment of display information for multiple types of medical information may also be performed according to any of the various methods illustrated in the first to fourth embodiments.

Next, the CPU 12 determines whether an instruction to select at least one of the columns (one or more vertical columns and one or more horizontal rows) in the matrix display section 30 displayed on the display unit 17 in S61 has been input (S62). If an instruction to select a column has not been input (S62: NO), the process proceeds to the determination in S77. As shown in FIG. 19, the user can select a column in the matrix display section 30, either a vertical or horizontal row, for which they want to check the information in detail by operating the operation unit 16. As one example, in this embodiment, the user can input a column selection instruction by operating the operation unit 16 to display a column indication frame 65 on the column for which they want to check the information in detail. In this embodiment, the user can select either a vertical column or a horizontal row. In the example shown in FIG. 19, among the multiple vertical columns displayed for each acquisition time, the vertical column for the acquisition time "2021.5.6" is selected. In S62, the CPU 12 may also allow the user to select at least one of the multiple horizontal rows acquired for each type.

When a column selection instruction is input (S62: YES), the CPU 12 extracts the medical information that was displayed in the column selected in S62 from among the medical information of the target patient stored in the storage device 13, and displays the extracted medical information on the display unit 17 (S63). In this embodiment, when a vertical column is selected in S62, multiple types of medical information corresponding to the acquisition time of the selected vertical column are displayed on the display unit 17. Also, when a horizontal row is selected in S62, the type of medical information of the selected horizontal row (the medical information acquired at each of multiple acquisition times) is displayed on the display unit 17.

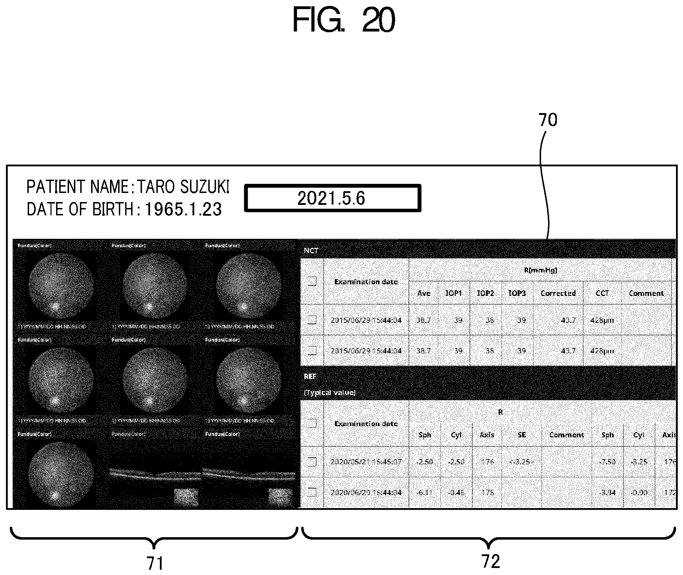

FIG. 20 is a diagram showing an example of the column information display section 70 that is displayed when the column for the acquisition time "2021.5.6" in the matrix display section 30 of FIG. 19 is selected by the user. As shown in FIG. 20, when a column for a specific acquisition time (in this embodiment, a vertical column) is selected in S62, multiple types of medical information corresponding to the acquisition time of the selected column are displayed in the column information display section 70. As shown in FIG. 20, in S63, the CPU 12 displays at least some of the multiple types of medical information corresponding to the selected column in the column information display section 70 as information that is more detailed or enlarged than the simplified information that was displayed in the matrix display section 30 (see FIG. 19). In the example shown in FIG. 20, for "intraocular pressure," not only the average value of multiple measurements for one eye is displayed, but each of the individual measured values is also shown. In addition to representative images among the multiple captured images, each of the multiple captured images is also displayed. Therefore, by selecting the column for which they wish to check information, the user can identify the medical information of the selected column in greater detail than the information that was displayed in the matrix display section 30. As a result, the necessary medical information is presented to medical professionals even more appropriately.

In S63 of this embodiment, the CPU 12 transitions the display style from the matrix display section 30 (see FIG. 19) that was displayed on the display unit 17 to the column information display section 70 (see FIG. 20), which displays the medical information of the column that was displayed in the column selected in S62. Accordingly, by having the display style transition from the matrix display section 30 to the column information display section 70, the user can more easily recognize that the information displayed on the display screen of the display unit 17 has transitioned to the medical information of the selected column. Furthermore, the CPU 12 can also display the medical information of the selected column in the column information display section 70 using a method different from the display method in the matrix display section 30. For example, it is possible to display more detailed information than the information in the matrix display section 30, and to display numerical information and image information separately (as will be described later), or by at least one of these methods.

As shown in FIG. 20, when multiple types of medical information corresponding to the selected column comprise both image information and numerical information, the CPU 12 displays an image information display section 71 for displaying image information and a numerical information display section 72 for displaying numerical information separately. Accordingly, the user can more easily and appropriately identify each of the image information and numerical information.

In this embodiment, in S62, the CPU 12 can also accept instructions to select two or more vertical columns among the multiple vertical columns in the matrix display section 30, or two or more horizontal rows among the multiple horizontal rows. When multiple columns are selected in S62, in S63, the CPU 12 extracts, from the target patient's medical information, the types or acquisition times of medical information that were displayed in the two or more selected columns (whether vertical or horizontal) in the matrix display section 30, and displays them in the column information display section 70. Therefore, if there are multiple columns that the user wishes to check, the user can easily confirm the medical information of the multiple selected columns simply by selecting them. As a result, necessary medical information is appropriately presented to medical professionals.

Returning to the explanation in FIG. 18. In this embodiment, when multiple types of medical information corresponding to the selected column comprise both image information and numerical information, the user can operate the operation unit 16 to input an instruction to the medical information processing device 10 to display only one of either the image information or the numerical information. When an instruction to display only one of the image information or numerical information is input (S65: YES), the CPU 12 extracts only either the image information or the numerical information, according to the instruction input in S65, from among the multiple types of medical information displayed in S63, and displays it on the display unit 17. Accordingly, the user can further identify in detail only one of either the image information or the numerical information among the multiple types of medical information corresponding to the selected column.

In this embodiment, when the multiple types of medical information corresponding to the selected column comprise both the medical information of the left tissue (in this embodiment, the left eye) and the right tissue (in this embodiment, the right eye) of the target patient, the user can operate the operation unit 16 to input an instruction to the medical information processing device 10 to display either the medical information of the left tissue or the right tissue. When an instruction to display either the medical information of the left tissue or the right tissue is input (S68: YES), the CPU 12 extracts only the medical information of either the left tissue or the right tissue, according to the instruction input in S68, from among the multiple types of medical information displayed in S63, and displays it on the display unit 17. Accordingly, the user can further identify in detail only the medical information of either the left tissue or the right tissue among the multiple types of medical information corresponding to the selected column.

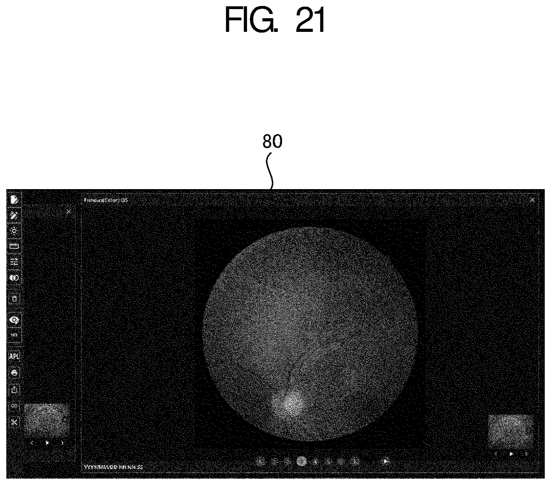

In this embodiment, when the multiple types of medical information corresponding to the selected column comprise image information, the user can operate the operation unit 16 to input an instruction to select specific image information among those displayed in the column information display section 70. When an instruction to select specific image information comprised in the column information display section 70 is input (S71), the CPU 12 enlarges and displays the selected image information, making it larger than the image previously displayed in the column information display section 70 (S72). Furthermore, the CPU 12 executes processing on the image information that is enlarged and displayed in S72, according to instructions input by the user (for example, at least one of image enlargement processing, reduction processing, extraction processing, drawing processing on the image, various numerical measurement processing, compositing processing, or transfer processing to other devices) (S73). FIG. 21 shows an example of the drawing information processing screen 80 displayed on the display unit 17 in S72. On the drawing information processing screen 80, the image is displayed in an enlarged form compared to the image shown in the column information display section 70, and multiple buttons for executing various operations on the image information are displayed alongside the image. Accordingly, by selecting the desired image information, the user can have the selected image information displayed in an enlarged view and perform various operations on it. As a result, necessary medical information is appropriately presented to medical professionals.

Next, the CPU 12 determines whether an instruction has been input to return the display from the column information display section 70 to the matrix display section 30 (S75). If the instruction has not been input (S75: NO), the process returns to S65, and the processing from S65 to S75 is repeated. When an instruction to return to the matrix display section 30 is input (S75: YES), the display on the display unit 17 is switched from the column information display section 70 back to the matrix display section 30, and the processing of S62, S77, and S79 is repeated.

In S77, the CPU 12 determines whether an instruction has been input by the user to select at least one of the multiple cells arranged in a grid within the matrix display section 30 (see FIG. 19). When the operation unit 16 is operated and an instruction to select a specific cell is input (S77: YES), the CPU 12 displays the medical information of the selected cell on the display unit 17 as information that is more detailed or enlarged than the simplified information that was displayed in the matrix display section 30 (S78). Accordingly, by selecting the cell for which they wish to check information, the user can identify the medical information of the selected cell in greater detail than the information that was displayed in the matrix display section 30. As a result, the necessary medical information is presented to medical professionals even more appropriately. If a cell containing image information is selected in S77, the CPU 12 may perform enlarged display of the image, as in S72, and may also execute various processing on the image, as in S73, for the image information of the selected cell. If an instruction to terminate processing has not been input (S79: NO), the process returns to S62. When an instruction to terminate processing is input (S79: YES), the CPU 12 ends the display processing of information for the target patient specified in S2, and the process returns to S1.

The technology disclosed in the above embodiments is merely an example. Therefore, it is also possible to modify the techniques illustrated in the above embodiments. First, it is possible to execute only a part of the techniques exemplified in the above embodiments. For example, in the third embodiment, either graph 60 or the difference information (difference icon 51) alone may be displayed as transition information on the display unit 17. Additionally, it is possible to combine multiple techniques illustrated in different embodiments among the first to fifth embodiments. For example, both the disease history information exemplified in the second embodiment and the value transition information (at least one of graph 60 or the difference information) exemplified in the third embodiment may be displayed together on the display unit 17.

## Claims

1. A medical information processing program executed in a medical information processing device that processes medical information concerning a patient, a database configured to store, for each patient, a plurality of types of medical information in association with acquisition time of each piece of information, the medical information processing program, when executed by a control unit of the medical information processing device, causing the medical information processing device to execute:
a patient identification step of identifying a target patient whose medical information is to be processed; and
a matrix display step of displaying, as a matrix display on a display unit, at least some of the plurality of types of medical information concerning the target patient by arranging the plurality of types of medical information by type in one of a vertical column and a horizontal row and by arranging the information by the acquisition time in chronological order in an other of the vertical column and the horizontal row.

2. The medical information processing program according to claim 1, further causing the medical information processing device to execute
a display rule setting step of setting at least one display rule from among a plurality of display rules, wherein the plurality of display rules includes at least one of a rule for narrowing down the types of medical information to be displayed on the display unit from the plurality of types of medical information concerning the target patient and a rule for determining a display order of the plurality of types of medical information, wherein
in the matrix display step, the medical information processing program further causes the medical information processing device to:
execute, for the plurality of types of medical information concerning the target patient, at least one of a narrowing process for the types and a determination process for the display order according to the display rule set in the display rule setting step; and then
display, on the display unit, the plurality of types of medical information by arranging the plurality of types of medical information in the display order in one of the vertical column and the horizontal row and by arranging the information in the chronological order of the acquisition time in the other of the vertical column and the horizontal row.

3. The medical information processing program according to claim 2, further causing the medical information processing device to execute
a display rule generation step of generating a display rule according to an instruction input by a user and storing the generated display rule in the database.

4. The medical information processing program according to claim 2, wherein
in the display rule setting step, at least one display rule is set from among the plurality of display rules according to an instruction input by a user.

5. The medical information processing program according to claim 2, wherein
at least one of a rule for narrowing down the plurality of types of medical information and a rule for determining the display order, the plurality of display rules includes a plurality of rules each defined according to a respective one of a plurality of diseases, and
in the display rule setting step, one display rule of the plurality of display rules corresponding to a disease of the target patient is set.

6. The medical information processing program according to claim 5, further causing the medical information processing device to execute
a disease information acquisition step of acquiring disease information concerning a disease of the target patient, wherein
in the display rule setting step, one display rule of the plurality of display rules corresponding to the disease indicated by the disease information of the target patient acquired in the disease information acquisition step is automatically set.

7. The medical information processing program according to claim 6, wherein
in the disease information acquisition step, the disease information of the target patient is acquired by inputting the medical information of the target patient into a mathematical model, and
the mathematical model is trained according to a machine learning algorithm to output an automatic determination result of a disease of a patient when medical information of the patient is input.

8. The medical information processing program according to any one of claims 5 to 7, further causing the medical information processing device to execute
a disease history information display step of displaying, on the display unit, disease history information indicating a course of a specific disease of the target patient in chronological order, based on medical information concerning the specific disease of the target patient.

9. The medical information processing program according to any one of claims 1 to 8, further causing the medical information processing device to execute
a value transition information display step of displaying, on the display unit together with the matrix display, value transition information indicating a transition of values over time for at least some types of medical information whose values are variable among the plurality of types of medical information displayed in the matrix display step.

10. The medical information processing program according to claim 2, further causing the medical information processing device to execute
a status information acquisition step of acquiring status information regarding at least one of a usage status of the medical information processing device and a status of the target patient, wherein
in the display rule setting step, the rule for narrowing down the types of the medical information to be displayed on the display unit from the plurality of types of medical information concerning the target patient and the rule for determining the display order of the plurality of types of medical information are set based on the status information acquired in the status information acquisition step, and
in the matrix display step:
at least one of the narrowing process for the types and the determination process for the display order is executed for the plurality of types of medical information concerning the target patient; and then
according to the display rule set in the display rule setting step, display, on the display unit as the matrix display, the plurality of types of medical information by arranging the plurality of types of medical information in the display order in one of the vertical column and the horizontal row and by arranging the information in chronological order of acquisition time in the other of the vertical column and the horizontal row.

11. The medical information processing program according to any one of claims 1 to 10, further causing the medical information processing device to execute:
a column/row selection reception step of receiving an instruction to select at least one column/row among one or more vertical columns and one or more horizontal rows in the matrix display; and
a column/row selection reception step of:
when the column/row selected in the column/row selection reception step is a vertical column, extracting, from the medical information of the target patient stored in the database, the type or the acquisition time of the medical information displayed in the selected vertical column and displaying the type or the acquisition time on the display unit; and
when the column/row selected in the column/row selection reception step is a horizontal row, extracting, from the medical information of the target patient stored in the database, the type or the acquisition time of the medical information displayed in the selected horizontal row and displaying the type or the acquisition time on the display unit.

12. The medical information processing program according to claim 11, wherein
in the matrix display step, simplified information obtained by excerpting part of information or reducing image information for at least one of the plurality of types of medical information concerning the target patient is displayed as the matrix display on the display unit, and
in the column/row selection reception step, at least one of the plurality of medical information concerning the target patient is displayed on the display unit as information more detailed or enlarged than the simplified information displayed in the matrix display.

13. The medical information processing program according to claim 11 or 12, wherein
in the column/row selection reception step, a display style is transitioned from a matrix display section displayed on the display unit in the matrix display step to a column information display section that displays the medical information displayed in the column/row selected in the column/row selection reception step.

14. The medical information processing program according to claim 12, further causing the medical information processing device to execute:
a cell selection reception step of receiving an instruction to select at least one cell among a plurality of cells arranged in a grid in the matrix display; and
a cell information display step of displaying, on the display unit, the medical information of the cell selected in the cell selection reception step as information more detailed or enlarged than the simplified information displayed in the matrix display.

15. The medical information processing program according to any one of claims 11 to 14, wherein
in the column/row selection reception step, an instruction to select two or more vertical columns among the plurality of vertical columns in the matrix display or two or more horizontal rows among the plurality of horizontal rows in the matrix display is received, and
when two or more columns/rows are selected in the column/row selection reception step, in the column/row selection reception step, the type or the acquisition time of the medical information displayed in the two or more selected columns/rows among the vertical columns and the horizontal rows in the matrix display is extracted from the medical information of the target patient stored in the database and displayed on the display unit.

16. The medical information processing program according to any one of claims 11 to 15, further causing the medical information processing device to execute
when the plurality of medical information displayed in the column/row selection reception step includes both image information and numerical information, a re-extraction display step of extracting and displaying, on the display unit, only one of the image information or the numerical information from the plurality of medical information according to an instruction input by a user.

17. The medical information processing program according to any one of claims 11 to 16, wherein
the database is configured to store, among tissues present on left and right sides of a human body, the medical information of both a left tissue and a right tissue of each patient, and
when the plurality of medical information displayed in the column/row selection reception step includes the medical information of the left tissue and the medical information of the right tissue, the medical information processing program further causes the medical information processing device to execute a left-right extraction display step of extracting and displaying, on the display unit, only one of the medical information of the left tissue and the medical information of the right tissue from the plurality of medical information according to an instruction input by the user.

18. The medical information processing program according to any one of claims 11 to 17, further causing the medical information processing device to execute:
an image selection reception step of receiving an instruction to select at least one image information among the plurality of medical information displayed in the matrix display step or the column/row selection reception step;
an enlargement display step of displaying the image information selected in the image selection reception step in an enlarged manner compared to the image displayed in the matrix display step or the column/row selection reception step; and
an image processing step of executing processing according to an instruction input by a user for the image information enlarged and displayed in the enlargement display step.

19. A medical information processing device that processes medical information concerning a patient, a database configured to store, for each patient, a plurality of types of medical information in association with acquisition time of each piece of information, the medical information processing device comprising
a control unit configured to execute:
a patient identifying step of identifying a target patient whose medical information is to be processed; and
a matrix display step of displaying, as a matrix display on a display unit, at least some of the plurality of types of medical information concerning the target patient by arranging the plurality of types of medical information by type in one of a vertical column or a horizontal row and by arranging the information by the acquisition time in chronological order in an other of the vertical column and the horizontal row.
